# EUROPEAN PATENT APPLICATION

(11) **EP 3 964 206 A1**
(43) Date of publication of application: **09.03.2022**
(21) Application number: 20194693.6
(22) Date of filing: 04.09.2020
(51) Int. Cl.: A61K 31/195, A61K 31/35, A61K 31/365, A61K 31/37, A61K 31/415, A61K 31/437, A61K 31/472, A61K 31/496, A61K 31/497, A61K 31/517, A61K 31/519, A61K 31/58, A61K 31/69, A61K 31/704, A61K 31/7076

(54) **COMPOUNDS FOR THE TREATMENT OF COVID-19**

(71) Applicant: Dompe' Farmaceutici S.P.A., 20122 Milan (IT); Fraunhofer Gesellschaft zur Förderung der angewandten Forschung e.V., 80686 München (DE); Katholieke Universiteit Leuven KU Leuven Research & Development, 3000 Leuven (BE)
(72) Inventor: BECCARI, Andrea Rosario, 80131 Napoli (IT); TALARICO, Carmine, 80131 Napoli (IT); MANELFI, Candida, 80131 Napoli (IT); Iaconi, Daniela, 80131 Napoli (IT); ZALIANI, Andrea, 22359 Hamburg (DE); GRIBBON, Philip, 22587 Hamburg (DE); GEISSLINGER, Gerd, 60596 Frankfurt am Main (DE); Claussen, Carsten, 80686 München (DE)
(74) Representative: Mauri, Elisabetta Maria Ester

(57) **Abstract**

The present invention relates to compounds for the treatment of a SARS-Cov-2 infection in a subject.

## Description

### FIELD OF THE INVENTION

The present invention relates to compounds that are able to inhibit replication of SARS-CoV-2 virus and thus are useful in the treatment of SARS-CoV-2 virus infections.

### STATE OF THE ART

Coronaviruses (Covs) are a large family of viruses belonging to the family Coronaviridae. The limited number of coronaviruses known to be circulating in humans were considered to cause mild infections and they were regarded in the past as relatively harmless respiratory human pathogens. However, in the last years the emergence of the severe acute respiratory syndrome coronavirus (SARS-CoV) and the Middle East Respiratory Syndrome (MERS) virus revealed that some coronaviruses can cause severe and sometimes fatal respiratory tract infections in humans (Pereira, H et al., 1989 Coronaviridae. Andrewes 'Viruses of Vertebrates, 5th ed.pp. 42-57; Holmes, K.V. et al., 1996. Virology 1, 1075-1093). The first known case of SARS-CoV occurred in Foshan, China in November 2002 and new cases emerged in mainland China in February 2003. The first emergence of MERS-CoV occurred in June 2012 in Saudi Arabia. These events demonstrated that the threats of CoVs should not be underestimated and that it is of paramount importance to advance the knowledge on the replication of these viruses and their interactions with the hosts to develop treatments and vaccines.

In December 2019, atypical pneumonia cases emerged in China and the cause was identified as being a novel coronavirus named SARS-CoV-2 by WHO.

Investigations of the epidemiological and clinical characteristics, and outcomes of patients infected by SARS-CoV-2 demonstrated that the infection caused clusters of severe respiratory illness similar to the known SARS-CoV. Furthermore, it was demonstrated that the SARS-CoV-2 can cause severe illness in some patients, even initially it did not transmit readily between people. However, more recent epidemiological data suggest the new virus has undergone human host adaptation/evolution and has become more efficient in human-to-human transmission. Analysis of SARS-CoV-2 genome sequences obtained from patients during the beginning of the outbreak demonstrated that they are almost identical to each other and share 79.5% sequence identity to SARS-CoV. Furthermore, the SARS-CoV-2 is 96% identical at the whole genome level to a bat coronavirus. Phylogenetic analysis of CoVs of different species indicated that SARS-CoV-2 could have originated from Chinese horseshoe bats, but the intermediate transmission vehicle has not yet been identified (Dong, N. et al. Microbiology 2020). According to this study, SARS-CoV-2 belongs to a novel type of bat coronavirus owing to a high degree of variation from the human SARS virus. SARS-CoV-2 is the seventh member of the family of CoVs that infect humans.

Like SARS-CoV, SARS-CoV-2 enters target cells through an endosomal pathway and also uses the same cell entry receptor, Angiotensin-converting enzyme II (ACE2).

An effective therapeutic targets in SARS-CoV and several other CoVs, are replication-related enzymes, such as proteases (Zhou, P. et al. Microbiology 104, 2020). Drugs that inhibit conserved proteases are capable of preventing replication and proliferation of the virus by interfering with the post-translational processing of essential viral polypeptides. They can also reduce the risk of mutation-mediated drug resistance. This was the case for the SARS-CoV, as inhibitors targeting the main protease involved in replication and proliferation were the most effective means to alleviate the epidemic.

However, the design and development of new drugs is a lengthy process that is not thus adequate to face the emergency of the immediate global challenge of COVID-19 outbreak.

An alternative, more efficient approach is the repurposing of drugs already tested as safe in man or approved for different therapeutic applications. This is a rapid response solution, since the pharmacokinetic, toxicological, and manufacturing data for the drigs are already available, thus allowing immediate application in clinical setting. 2006;6(4):361-76) (Anand et al., Science 2003;300(5626):1763-7).

Therefore, computational drug repurposing is an effective approach to rapidly identify and select drugs safe in man that are promising candidates in the treatment of SARS-Cov-2

### SUMMARY OF THE INVENTION

The applicants have carried out the analysis of a library containing more than 10.000 commercialized drugs and clinical candidates "safe in man" or characterized up to late clinical stage and have selected by Computer-Aided Drug Design a number of molecules able to bind to SARS-Cov-2 functional proteins essential for the pathogenetic mechanisms. Furthermore, the applicants have confirmed by in vitro screening the ability of the selected molecules to inhibit virus survival and replication in infected host cells.

In details, the compounds selected are better described below, wherein also the chemical name and formula for each compound are provided.

| **N.** | **Chemical structure** | **Generic/Drug Name** | **Chemical Name** |
|---|---|---|---|
| 1 | 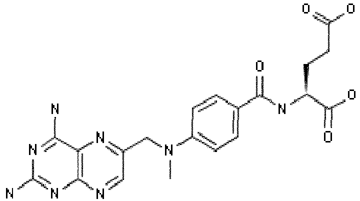 | METHOTREXATE | N-[4-[N-(2,4-Diaminopteridin-6-ylmethyl)-N-methylamino]benzoyl]-L-glutamic acid |
| 2 | 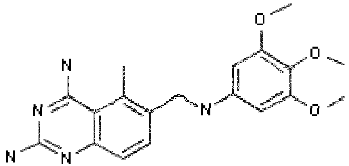 | Trimetrexate | 2,4-Diamino-5-methyl-6-[(3,4,5-trimethoxyanilino)methyl]quina zoline 5-Methyl-6-[[(3,4,5-trimetoxyphenyl)amino]methyl ]-2,4-quinazolinediamine |
| 3 | 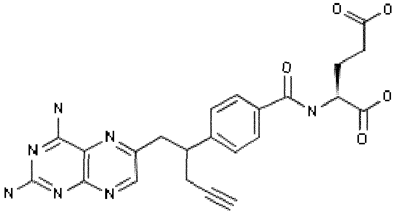 | Pralatrexate | N-[4-[2-(2,4-Diaminopteridin-6-yl)-1-(2-propynyl)ethyl]benzoyl]-L-glutamic acid 10-Propargyl-10-deazaaminopterin |
| 4 | 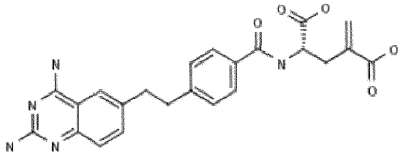 | CH-4051 | N-[4-[2-(2,4-Diaminoquinazolin-6-yl)ethyl]benzoyl]-4-methylene-L-glutamic acid |
| 5 | 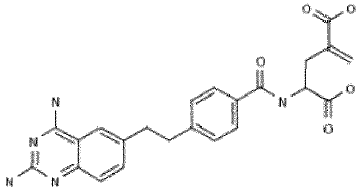 | CH-1504 | N-[4-[2-(2,4-Diaminoquinazolin-6-yl)ethyl]benzoyl]-4-methylene-DL-glutamic acid |
| 6 | 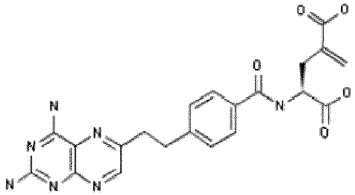 | L-MDAM | N-[4-[2-(2,4-Diaminopteridin-6-yl)ethyl]benzoyl]-4-methylene-L-glutamic acid L-gamma-Methylene-10-deazaaminopterin |
| 7 | 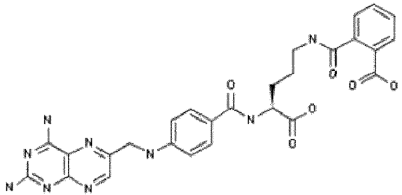 | Talotrexin | Nalpha-(4-Amino-4-deoxypteroyl)-Ndeltahemiphthaloyl-L-ornithine |
| 8 | 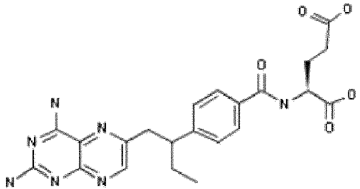 | Edatrexate | 10-Ethyl-10-deazaaminopterin N-[4-[1-(2,4-Diamino-6-pteridinylmethyl)propyl]benzoy l]-L-glutamic acid |
| 9 | 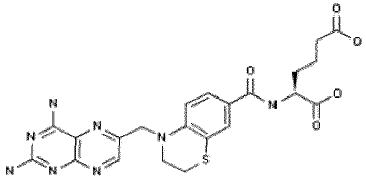 | MX-68 | N-[4-(2,4-Diaminopteridin-6-ylmethyl)-3,4-dihydro-2H-1,4-benzothiazin-7-ylcarbonyl]-L-(3'-homo)glutamicacid 2(S)-[4-(2,4-Diaminopteridin-6-ylmethyl)-3,4-dihydro-2H-1,4-benzothiazin-7-ylcarboxamido]adipic acid |
| 10 | 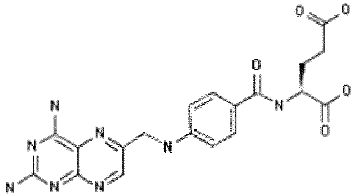 | Aminopterin | N-[4-(2,4-diamino-6-pteridinylmethylamino)benzolyl ]-L-glutamic acid |
| 11 | 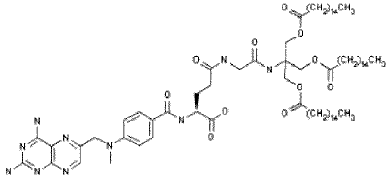 | MTX-gamma-GTP-3 | Nalpha-[4-[N-(2,4-Diamino-6-pteridinylmethyl)-N-methylamino]benzoyl]-Ndelta-[2-[2-(hexadecanoyloxy)-1,1-bis(hexadecanoyloxymethyl)eth ylamino]-2-oxoethyl]-L-glutamine |
| 12 | 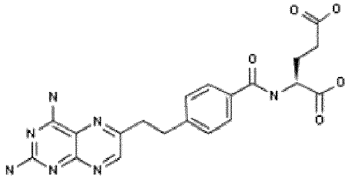 | 10-Deazaaminopteri n | N-[4-[2-(2,4-Diaminopteridin-6-yl)ethyl]benzoyl]-L-glutamic acid |
| 13 | 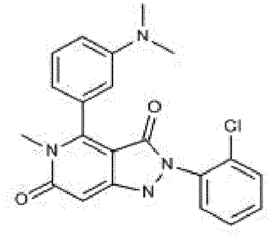 | Setanaxib | 2-(2-Chlorophenyl)-4-[3-(dimethylamino)phenyl]-5-methyl-1H-pyrazolo[4,3-c]pyridine-3,6(2H,5H)-dione |
| 14 | 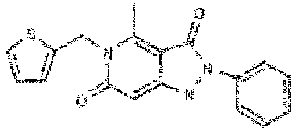 | 640486 | 4-Methyl-2-phenyl-5-(thien-2-ylmethyl)-2,3,5,6-tetrahydro-1H-pyrazolo[4,3-c]pyridine-3,6-dione |
| 15 | 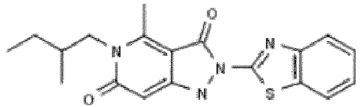 | 640491 | 2-(1,3-Benzothiazol-2-yl)-4-methyl-5-(2-methylbutyl)-2,3,5,6-tetrahydro-1H-pyrazolo[4,3-c]pyridine-3,6-dione |
| 16 | 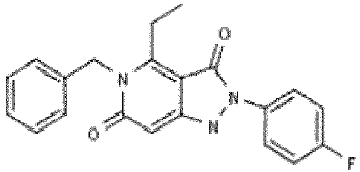 | 640493 | 5-Benzyl-4-ethyl-2-(4-fluorophenyl)-2,3,5,6-tetrahydro-1H-pyrazolo[4,3-c]pyridine-3,6-dione |
| 17 | 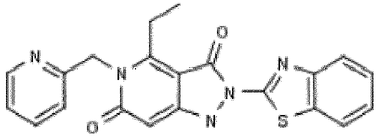 | 640497 | 2-(1,3-Benzothiazol-2-yl)-4-ethyl-5-(pyridin-2-ylmethyl)-2,3,5,6-tetrahydro-1H-pyrazolo[4,3-c]pyridine-3,6-dione |
| 18 | 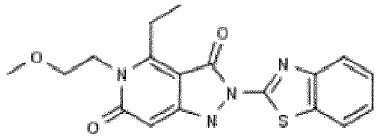 | 640498 | 2-(1,3-Benzothiazol-2-yl)-4-ethyl-5-(2-methoxyethyl)-2,3,5,6-tetrahydro-1H-pyrazolo[4,3-c]pyridine-3,6-dione |
| 19 | 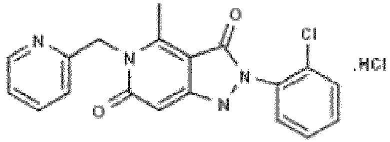 | GKT-136901 | 2-(2-Chlorophenyl)-4-methyl-5-(pyridin-2-ylmethyl)-2,3,5,6-tetrahydro-1H-pyrazolo[4,3-c]pyridine-3,6-dione hydrochloride |
| 20 | 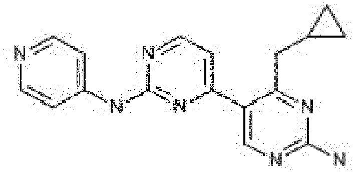 | PIK-III | 4'-(Cyclopropylmethyl)-N2-(pyridin-4-yl)-4,5'-bipyrimidine-2,2'-diamine |
| 21 | 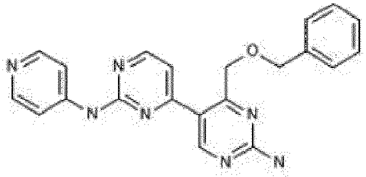 | 781398 | 4'-[(Benzyloxy)methyl]-N2-(pyridin-4-yl)-4,5'-bipyrimidine-2,2'-diamine |
| 22 | 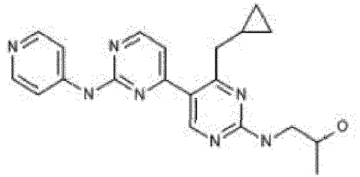 | 781406 | 1-[[4'-(Cyclopropylmethyl)-2-(pyridin-4-ylamino)-4,5'-bipyrimidin-2'-yl]amino]propan-2-ol |
| 23 | 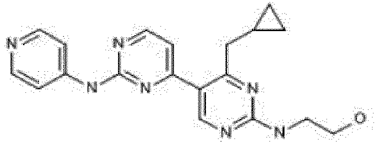 | 781408 | 2-[[4'-(Cyclopropylmethyl)-2-(pyridin-4-ylamino)-4,5'-bipyrimidin-2'-yl]amino]ethanol |
| 24 | 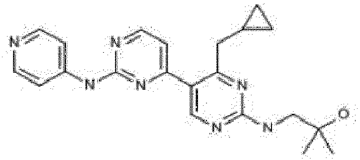 | 781410 | 1-[[4'-(Cyclopropylmethyl)-2-(pyridin-4-ylamino)-4,5'-bipyrimidin-2'-yl]amino]-2-methylpropan-2-ol |
| 25 | 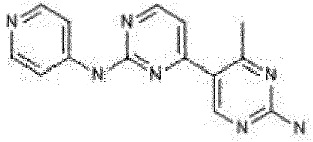 | 781424 | 4'-Methyl-N2-(pyridin-4-yl)-4,5'-bipyrimidine-2,2'-diamine |
| 26 | 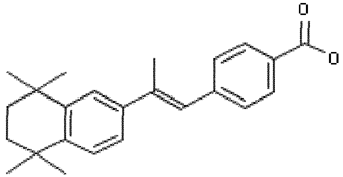 | Arotinoid acid | 4-[2-(5,5,8,8-Tetramethyl-5,6,7,8-tetrahydronaphthalen-2-yl)-1(E)-propenyl]benzoic acid |
| 27 | 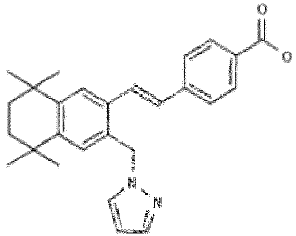 | Palovarotene | 4-[(E)-2-[5,5,8,8-Tetramethyl-3-(1H-pyrazol-1-ylmethyl)-5,6,7,8-tetrahydronaphthalen-2-yl]vinyl]benzoic acid |
| 28 | 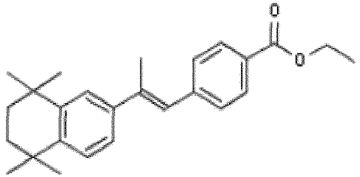 | Ro-136298 | p-[(E)-2-(5,6,7,8-Tetrahydro-5,5,8,8-tetramethyl-2-naphthyl)propenyl]benzoic acid ethyl ester (E)-4-[2-(5,6,7,8-Tetrahydro-5,5,8,8-tetramethyl-2-naphthalenyl)-1-propenyl]benzoic acid ethyl ester |
| 29 | 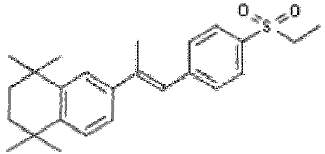 | Etarotene | Ethyl 4-[2(E)-(5,5,8,8-Tetramethyl-5,6,7,8-tetrahydro-2-naphthyl)propenyl]phenylsulfo ne |
| 30 | 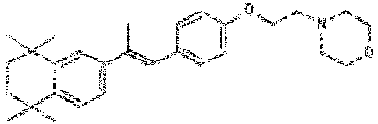 | Mofarotene | 4-[2-[4-[2(E)-(5,5,8,8-Tetramethyl-5,6,7,8-tetrahydro-2-naphthyl)-1-propenyl]phenoxy]ethyl]morph oline |
| 31 | 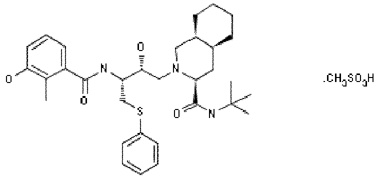 | Nelfinavir Mesilate | (3S,4aS,8aS)-N-tert-Butyl-2-[2(R)-hydroxy-3(R)-(3-hydroxy-2-methylbenzamido)-4-(phenylsulfanyl)butyl]perhydroi soquinoline-3-carboxamide methanesulfonate |
| 32 | 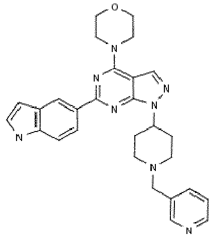 | WAY-600 | 6-(1H-Indol-5-yl)-4-(4-morpholinyl)-1-[1-(pyridin-3-ylmethyl)piperidin-4-yl]-1H-pyrazolo[3,4-d]pyrimidine |
| 33 | 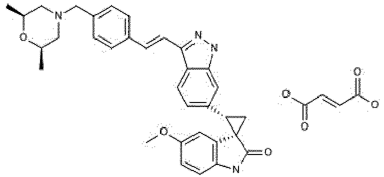 | CFI-400945 | (1R,2S)-2-[3-[(E)-2-[4-[(cis-2,6-Dimethylmorpholin-4-yl)methyl]phenyl]vinyl]-1H-indazol-6-yl]-5'-methoxyspiro[cyclopropane-1,3'-indol]-2'(1'H)-one (2E)-but-2-enedioate |
| 34 | 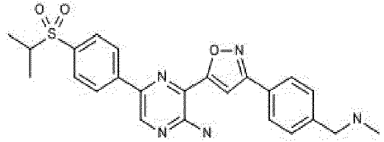 | VE-822 (Berzosertib) | 3-[3-[4-[(Methylamino)methyl]phenyl]-1,2-oxazol-5-yl]-5-[4-(propan-2-ylsulfonyl)phenyl]pyrazin-2-amine |
| 35 | 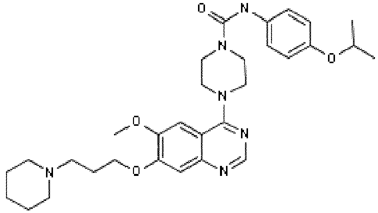 | Tandutinib | N-(4-Isopropoxyphenyl)-4-[6-methoxy-7-[3-(1-piperidinyl)propoxy]quinazolin-4-yl]piperazine-1-carboxamide |
| 36 | 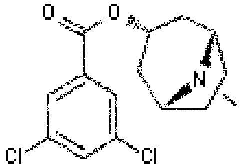 | Bemesetron | 1alphaH,3alpha,5alphaH-Tropan-3-yl-3,5-dichlorobenzoate; 3,5-Dichlorobenzoic acid endo-8-methyl-8-azabicyclo[3.2.1]oct-3-yl ester |
| 37 | 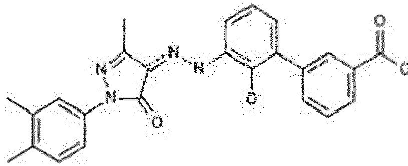 | Eltrombopag | 3'-[2(Z)-[1-(3,4-Dimethylphenyl)-3-methyl-5-oxo-4,5-dihydro-1H-pyrazol-4-ylidene]hydrazino]-2'-hydroxybiphenyl-3-carboxylic acid |
| 38 | 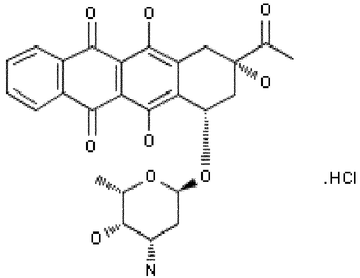 | Idarubicin hydrochloride | 4-Demethoxydaunorubicin hydrochloride; (1S,3S)-3-Acetyl-1,2,3,4,6,11-hexahydro-3,5,12-trihydroxy-6,11-dioxo-1-naphthacenyl 3-amino-2,3,6-trideoxy-alpha-L-lyxohexopyranoside hydrochloride; (7S,9S)-9-Acetyl-7-[(3-amino-2,3,6-trideoxy-alpha-L-lyxohexopyranosyl)oxy]-7,8,9,10-tetrahydro-6,9,11-trihydroxy-5,12-naphthacenedione hydrochloride; 4-Demethoxydaunomycin hydrochloride |
| 39 | 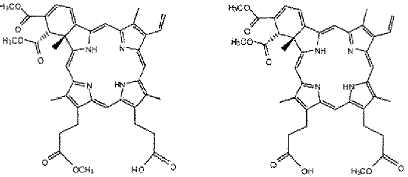 | Verteporfin | (4R,4aS)-18-Ethenyl-4,4a-dihydro-3,4-bis(methoxycarbonyl)-4a,8,14,19-tetramethyl-24H,26H-benzo[b]porphine-9,13-dipropanoic acid monomethyl ester |
| 40 | 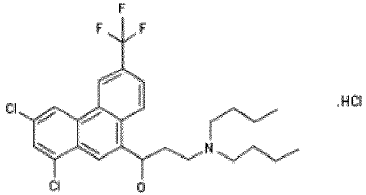 | Halofantrine hydrochloride | (±)-1,3-Dichloro-9-[3-(dibutylamino)-1-hydroxypropyl]-6-(trifluoromethyl)phenanthrene hydrochloride 3-(Dibutylamino)-1-[1,3-dichloro-6-(trifluoromethyl)phenanthren-9-yl]propan-1-ol |
| 41 | 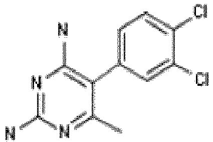 | Metoprine | 5-(3,4-Dichlorophenyl)-6-methylpyrimidine-2,4-diamine |
| 42 | 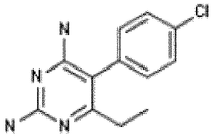 | Pyrimethamine | 5-(4-Chlorophenyl)-6-ethyl-2,4-pyrimidinediamine |
| 43 | 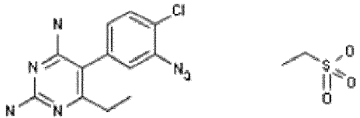 | MZPES | 5-(3-Azido-4-chlorophenyl)-6-ethyl-2,4-pyrimidine-diamine monoethanesulfonate |
| 44 | 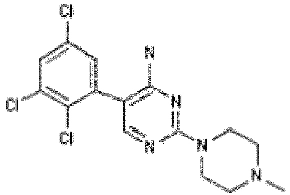 | Sipatrigine | 4-Amino-2-(4-methylpiperazin-1-yl)-5-(2,3,5-trichlorophenyl)pyrimidine 2-(4-Methylpiperazin-1-yl)-5-(2,3,5-trichlorophenyl)pyrimidine-4-amine |
| 45 | 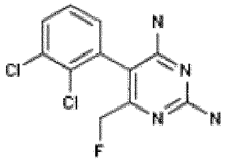 | BW-4030W92 | 5-(2,3-Dichlorophenyl)-6-(fluoromethyl)pyrimidine-2,4-diamine |
| 46 | 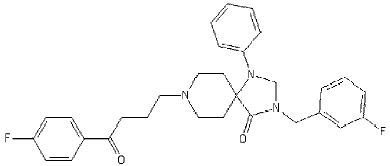 | 3'-fluorobenzylspip erone | 3-[(3-fluorophenyl)methyl]-8-[4-(4-fluorophenyl)-4-oxobutyl]-1-phenyl-1,3,8-triazaspiro[4.5]decan-4-one |
| 47 | 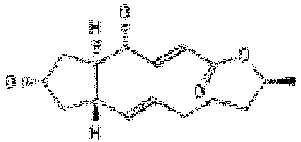 | brefeldin-a | (1R,2S,7S,13S,15S)-2,15-Dihydroxy-7-methyl-6-oxabicyclo[11.3.0]hexadeca-3(E),11(E)-dien-5-one [1S-(1R*,2E,6R*,10E,11aR*,13R*,14 aS*)]-1,6,7,8,9,11a,12,13,14,14a-Decahydro-1,13-dihydroxy-6-methyl-4H-cyclopent[f]oxacyclotridecin-4-one |
| 48 | 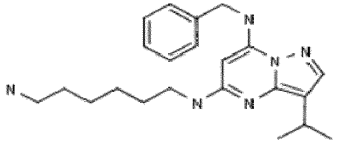 | BS-181 | N5-(6-Aminohexyl)-N7-benzyl-3-isopropylpyrazolo[1,5-a]pyrimidine-5,7-diamine |
| 49 | 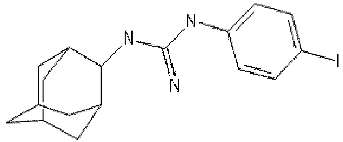 | IPAG | 2-(2-adamantyl)-1-(4-iodophenyl)guanidine |
| 50 | 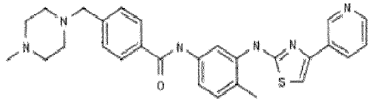 | Masitinib (AB1010) | 4-[(4-methylpiperazin-1-yl)methyl]-N-[4-methyl-3-[(4-pyridin-3-yl-1,3-thiazol-2-yl)amino]phenyl]benzamide |
| 51 | 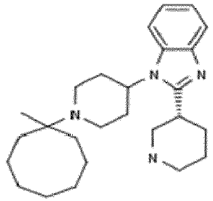 | MCOPPB | 1-[1-(1-Methylcyclooctyl)piperidin-4-yl]-2-[3(R)-piperidinyl]-1H-benzimidazole |
| 52 | 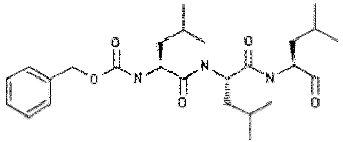 | MG-132 | Benzyloxycarbonyl-L-leucyl-L-leucyl-L-leucinal |
| 53 | 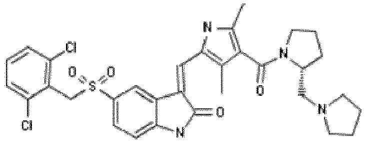 | PHA-665752 | 5-(2,6-Dichlorobenzylsulfonyl)-3(Z)-[3,5-dimethyl-4-[2(R)-(pyrrolidin-1-ylmethyl)pyrrolidin-1-ylcarbonyl]-1H-pyrrol-2-ylmethylene]-2,3-dihydro-1H-indol-2-one |
| 54 | 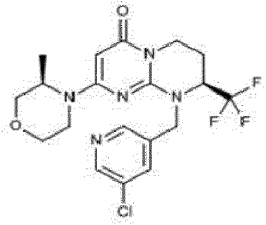 | SAR405 | (8S)-9-[(5-Chloropyridin-3-yl)methyl]-2-[(3R)-3-methylmorpholin-4-yl]-8-(trifluoromethyl)-6,7,8,9-tetrahydro-4H-pyrimido[1,2-a]pyrimidin-4-one |
| 55 | 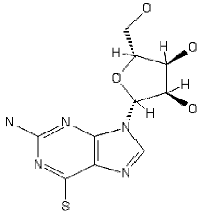 | Thioguanosine | 2-amino-9-[(2R,3R,4S,5R)-3,4-dihydroxy-5-(hydroxymethyl)oxolan-2-yl]-3H-purine-6-thione |
| 56 | 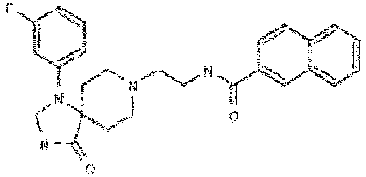 | VU-0364739 | N-[2-[1-(3-Fluorophenyl)-4-oxo-1,3,8-triazaspiro[4.5]dec-8-yl]ethyl]naphthalen-2-carboxamide |
| 57 | 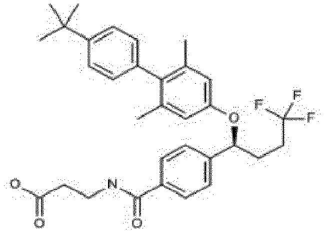 | ADOMEGLIVANT | N-(4-[(1S)-1-[(4'-tert-Butyl-2,6-dimethylbiphenyl-4-yl)oxy]-4,4,4-trifluorobutyl]benzoyl)-beta-alanine |
| 58 | 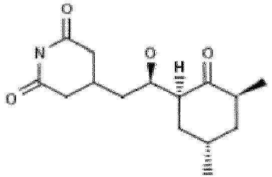 | CYCLOHEXIMIDE | 4-[2(R)-[(1S,3S,5S)-3,5-Dimethyl-2-oxocyclohexyl]-2-hydroxyethyl] piperidine-2,6-dione |
| 59 | 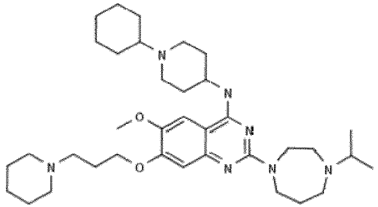 | UNC0646 | N-(1-Cyclohexylpiperidin-4-yl)-6-methoxy-7-[3-(1-piperidinyl)propoxy]-2-[4-(propan-2-yl)perhydro-1,4-diazepin-1-yl]quinazolin-4-amine |
| 60 | 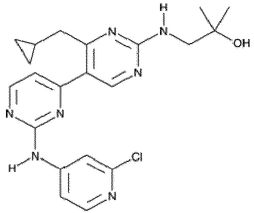 | VPS34-IN-1 | 1-[[2-[(2-chloro-4-pyridinyl)amino]-4'-(cyclopropylmethyl)[4,5'-bipyrimidin]-2'-yl]amino]-2-methyl-2-propanol |
| 61 | 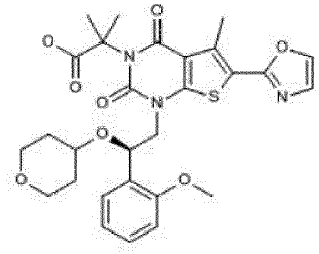 | N D-630 | 2-[1-[(2R)-2-(2-Methoxyphenyl)-2-(tetrahydro-2H-pyran-4-yloxy)ethyl]-5-methyl-6-(1,3-oxazol-2-yl)-2,4-dioxo-1,4-dihydrothieno[2,3-d]pyrimidin-3(2H)-yl]-2-methylpropanoic acid |
| 62 | 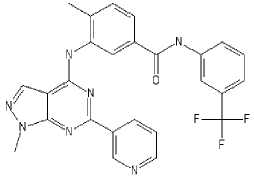 | NVP-BHG712 | 4-methyl-3-{[1-methyl-6-(3-pyridinyl)-1H-pyrazolo[3,4-d]pyrimidin-4-yl]amino}-N-[3-(trifluoromethyl)phenyl]benza mide |
| 63 | 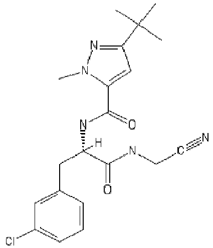 | Cathepsin Inhibitor 1 | 3-tert-butyl-N-{(1S)-1-(3-chlorobenzyl)-2-[(cyanomethyl)amino]-2-oxoethyl}-1-methyl-1H-pyrazole-5-carboxamide |
| 64 | 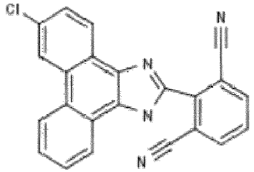 | MF63 | 2-(6-Chloro-1H-phenanthro[9,10-d]imidazol-2-yl)benzene-1,3-dicarbonitrile |
| 65 | 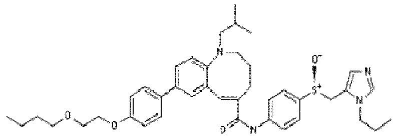 | CENICRIVIROC | (-)-(S)-8-[4-(2-Butoxyethoxy)phenyl]-1-isobutyl-N-[4-(1-propyl-1H-imidazol-5-ylmethylsulfinyl)phenyl]-1,2,3,4-tetrahydro-1-benzazocine-5-carboxamide |
| 66 | 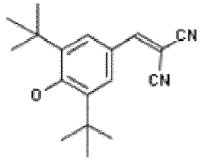 | TYRPHOSTIN A9 | (3,5-Di-tert-butyl-4-hydroxybenzylidene)malononitr ile |
| 67 | 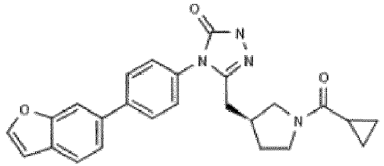 | GSK2194069 | 4-[4-(1-Benzofuran-6-yl)phenyl]-5-[1-(cyclopropylcarbonyl)pyrrolidin -3(S)-ylmethyl]-3,4-dihydro-2H-1,2,4-triazol-3-one |
| 68 | 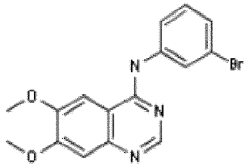 | PD153035 | 4-(3-Bromophenylamino)-6,7-dimethoxyquinazoline |
| 69 | 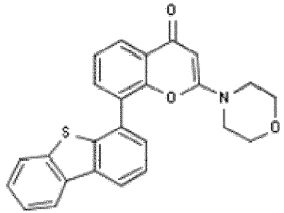 | NU 7441 | 8-(Dibenzo[b,d]thien-4-yl)-2-(4-morpholinyl)-4H-1-benzopyran-4-one |
| 70 | 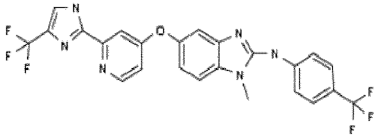 | CHIR-265 | 1-Methyl-N-[4-(trifluoromethyl)phenyl]-5-[2-[4-(trifluoromethyl)-1H-imidazol-2-yl]pyridin-4-yloxy]-1H-benzimidazol-2-amine |
| 71 | 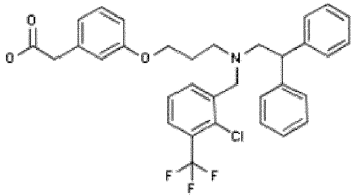 | GW3965 | 2-[3-[3-[N-[2-Chloro-3-(trifluoromethyl)benzyl]-N-(2,2-diphenylethyl)amino]propoxy]p henyl]acetic acid |
| 72 | 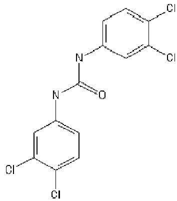 | MMV665852 | 1,3-Bis(3,4-dichlorophenyl)urea |
| 73 | 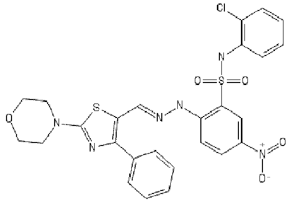 | PB49673382 | N-(2-chlorophenyl)-2-[(2E)-2-[(2-morpholin-4-yl-4-phenyl-1,3-thiazol-5-yl)methylidene]hydrazinyl]-5-nitrobenzenesulfonamide |
| 74 | 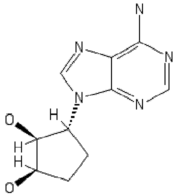 | DHCaA | (1R,2S,3R)-9-(2,3-Dihydroxycyclopentyl)adenine (1R,2S,3R)-3-(6-Amino-9H-purin-9-yl)-1,2-cyclopentanediol |
| 75 | 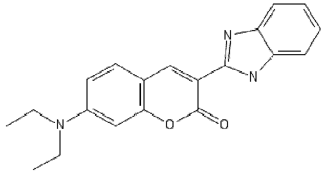 | Coumarin 7 | (1R,2S,3R)-3-(6-Amino-9H-purin-9-yl)-1,2-cyclopentanediol |
| 76 | 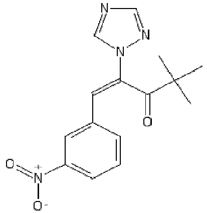 | Nexinhib20 | 4,4-dimethyl-1-(3-nitrophenyl)-2-(1H-1,2,4-triazol-1-yl)-1-penten-3-one |
| 77 | 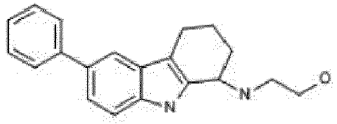 | Casin | 2-[(6-Phenyl-2,3,4,9-tetrahydro-1H-carbazol-1-yl)amino]ethanol |
| 78 | 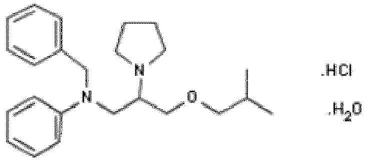 | Bepridil | 1-[1-(N-Benzylanilino)-3-isobutoxy-2-propyl]pyrrolidine monohydrochloride monohydrate |
| 79 | 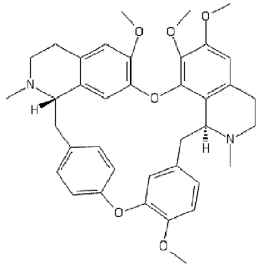 | Tetrandrine (Fanchinine) | (1beta)-(S,S)-6,6',7,12-Tetramethoxy-2,2'-dimethylberbaman |
| 80 | 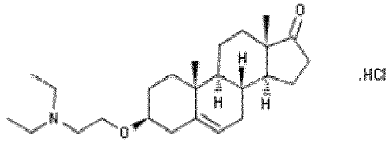 | U-18666° | 3beta-[2-(Diethylamino)ethoxy]androst-5-en-17-one hydrochloride |
| 81 | 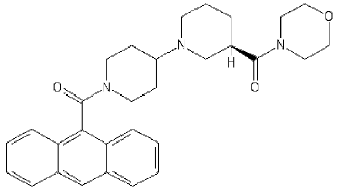 | CP-640186 | 1-[1'-(Anthracen-9-ylcarbonyl)-1,4'-bipiperidin-3(R)-yl]-1-(4-morpholinyl)methanone |
| 82 | 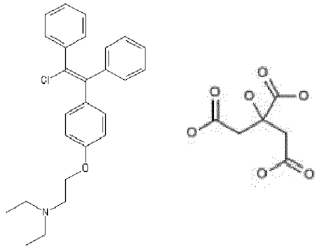 | CLOMIPHENE CITRATE | 2-[4-(2-Chloro-1,2-diphenylvinyl)phenoxy]-N,N-diethylethanamine citrate |
| 83 | 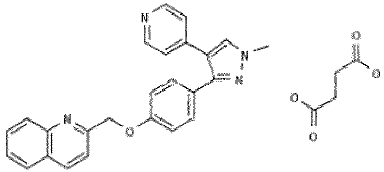 | PF-2545920 | 2-[4-[1-Methyl-4-(4-pyridyl)-1H-pyrazol-3-yl]phenoxymethyl]quinoline succinate |
| 84 | 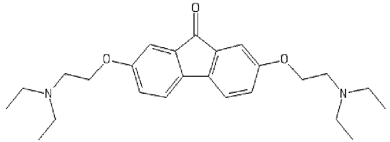 | TILORONE | 2,7-Bis[2-(diethylamino)ethoxy]-9H-fluoren-9-one |
| 85 | 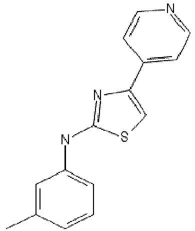 | STF-62247 | N-(3-Methylphenyl)-4-(4-pyridyl)thiazol-2-amine |
| 86 | 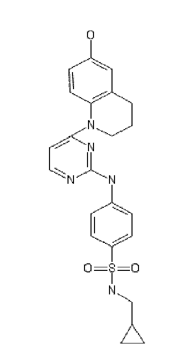 | Pyrintegrin | N-(Cyclopropyl methyl)-4-[[4-(3,4-dihydro-6-hydroxy-1(2 H)-quinolinyl)-2-pyrimidinyl]amino]benzenesulf onamide |
| 87 | 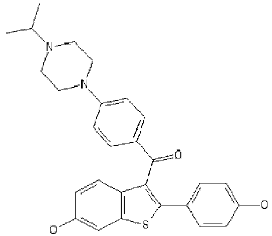 | Y-134 | 1-[6-Hydroxy-2-(4-hydroxyphenyl)-1-benzothien-3-yl]-1-[4-(4-isopropylpiperazin-1-yl)phenyl]methanone |
| 88 | 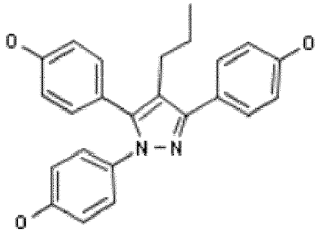 | PPT | 1,3,5-Tris(4-hydroxyphenyl)-4-propyl-1H-pyrazole |
| 89 | 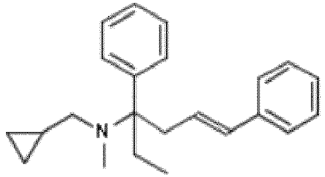 | Igmesine | (+)-N-(Cyclopropylmethyl)-N-[1-ethyl-1,4-diphenyl-3(E)-butenyl]-N-methylamine |
| 90 | 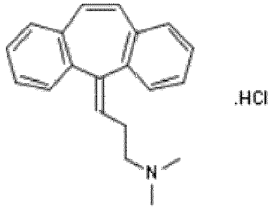 | Cyclobenzaprine Hydrochloride | 3-(5H-Dibenzo[a,d]cyclohepten-5-ylidene)-N,N-dimethylpropan-1-amine hydrochloride |
| 91 | 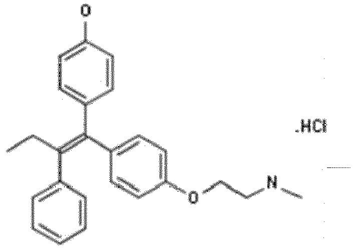 | Endoxifen Hydrochloride | 4-[1-[4-[2-(Methylamino)ethoxy]phenyl]-2-phenyl-1-butenyl]phenol hydrochloride |
| 92 | 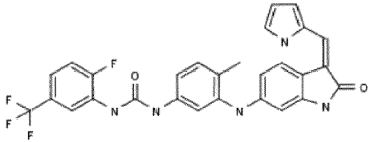 | GNF-5837 | 1-[2-Fluoro-5-(trifluoromethyl)phenyl]-3-[4-methyl-3-[2-oxo-3-(1H-pyrrol-2-ylmethylene)-2,3-dihydro-1H-indol-6-ylamino]phenyl]urea |
| 93 | 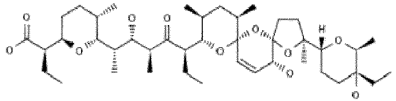 | Salinomycin | 2(R)-[6(R)-[5(R)-[(2S,5S,7R,9S,10S,12R,15R)-2-[5(R)-Ethyl-5-hydroxy-6(S)-methyltetrahydropyran-2(R)-yl]-15-hydroxy-2,10,12-trimethyl-1,6,8-trioxadispiro[4.1.5.3]pentadec-13-en-9-yl]-2(S)-hydroxy-1(S),3(S)-dimethyl-4-oxoheptyl]-5(S)-methyltetrahydropyran-2(R)-yl]butyric acid |
| 94 | 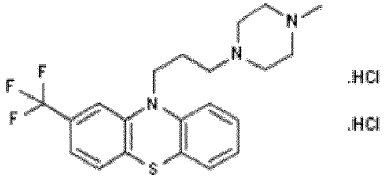 | Trifluoperazine dihydrochloride | 10-[3-(4-Methylpiperazin-1-yl)propyl]-2-(trifluoromethyl)-10H-phenothiazine dihydrochloride |
| 95 | 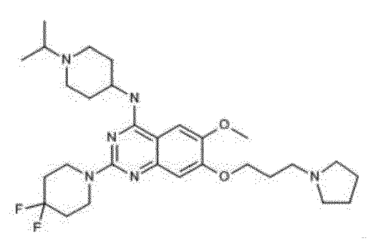 | UNC0642 | 2-(4,4-Difluoropiperidin-1-yl)-6-methoxy-N-[1-(propan-2-yl)piperidin-4-yl]-7-[3-(pyrrolidin-1-yl)propoxy]quinazolin-4-amine |
| 96 | 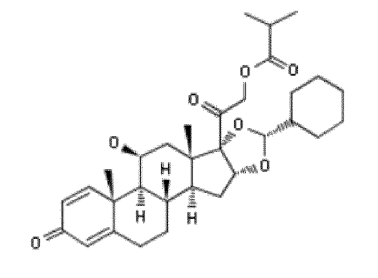 | Ciclesonide | 16alpha,17alpha-[(R)-Cyclohexylmethylenedioxy]-11beta-hydroxy-21-(isobutyryloxy)pregna-1,4-dien-3-one (R)-11beta,16alpha,17,21-Tetrahydroxypregna-1,4-diene-3,20-dione cyclic 16,17-acetal with cyclohexanecarboxaldehyde, 21-isobutyrate |
| 97 | 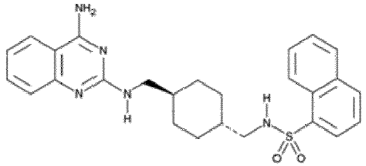 | CGP-71683 | N-[[trans-4-[[(4-Amino-2-quinazolinyl)amino]methyl]cycl ohexyl]methyl]-1-naphthalenesulfonamide |
| 98 | 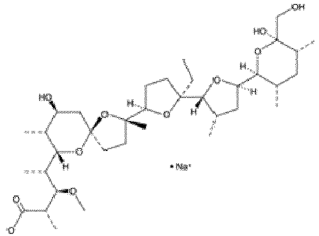 | Monensin Sodium | 4(S)-[(2S,5R,7S,8R,9S)-2-[(2S,2'R,3'S,5R,5'R)-2-Ethyl-5'-[6(R)-hydroxy-3(S),5(R)-dimethyl-6-(phenylaminocarbonyloxymeth yl)tetrahydropyran-2(S)-yl]-3'-methyl perhydro-2,2'-bifuran-5-yl]-9-hydroxy-2,8-dimethyl-1,6-dioxaspiro[4.5]dec-7-yl]-3(R)-methoxy-2(S)-methylpentanoic acid sodium salt |
| 99 | 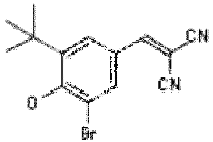 | AG1024 | 2-(3-Bromo-5-tert-butyl-4-hydroxybenzylidene)propanedi nitrile 2-(3-Bromo-5-tert-butyl-4-hydroxybenzylidene)malononitr ile |
| 100 | 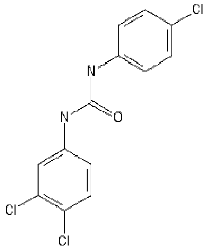 | Triclocarban | 3-(4-Chlorofenil)-1-(3,4-diclorofenil)urea |
| 101 | 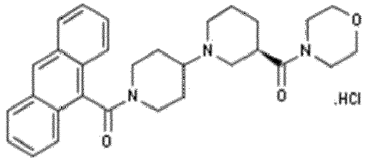 | CP-640186 hydrochloride | 1-[1'-(Anthracen-9-ylcarbonyl)-1,4'-bipiperidin-3(R)-yl]-1-(4-morpholinyl)methanone hydrochloride |
| 102 | 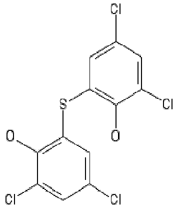 | Bithionol | 2,2'-Thio-bis(4,6-dichlorophenol), Bis(2-hydroxy-3,5-dichlorophenyl) sulfide |
| 103 | 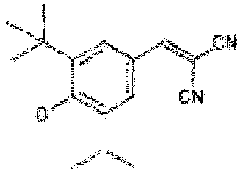 | Tyrphostin 9 | (3,5-Di-tert-butyl-4-hydroxybenzylidene)malononitr ile |
| 104 | 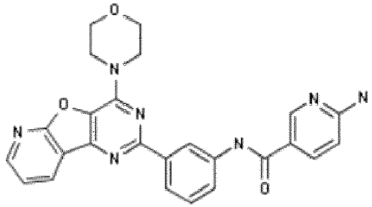 | YM201636 | 6-Amino-N-[3-[4-(4-morpholinyl)pyrido[3',2':4,5]fur o[3,2-d]pyrimidin-2-yl]phenyl]pyridine-3-carboxamide |
| 105 | 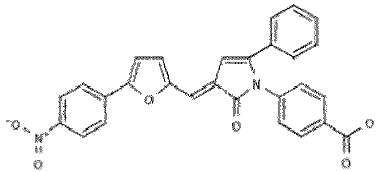 | 4E1RCat | 4-[3-[5-(4-Nitrophenyl)furan-2-ylmethylene]-2-oxo-5-phenyl-2,3-dihydro-1H-pyrrol-1-yl]benzoic acid |
| 106 | 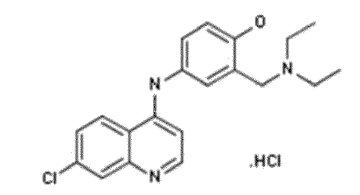 | Amodiaquine hydrochloride | 4-(7-Chloroquinolin-4-ylamino)-2-(diethylaminomethyl)phenol hydrochloride |
| 107 | 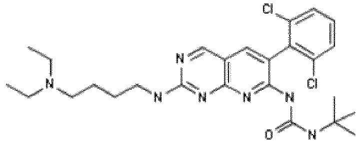 | PD-161570 | 1-tert-Butyl-3-[6-(2,6-dichlorophenyl)-2-[4-(diethylamino)butylamino]pyrid o[2,3-d]pyrimidin-7-yl]urea |
| 108 | 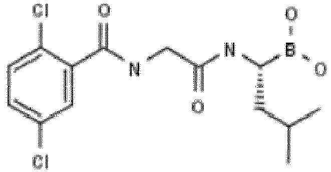 | Ixazomib | 1(R)-[2-(2,5-Dichlorobenzamido)acetamido] -3-methylbutylboronic acid |
| 109 | 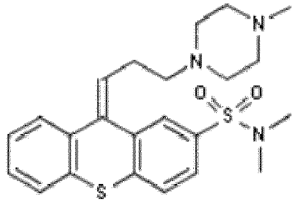 | Thiothixene | (Z)-N, N-Di methyl-9-[3-(4-methyl-1-piperazinyl)propylidene]thioxan thene-2-sulfonamide |
| 110 | 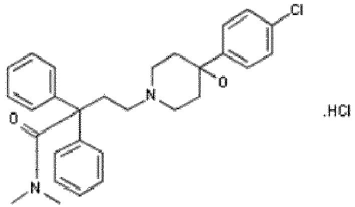 | Loperamide Hydrochloride | 4-[4-(4-Chlorophenyl)-4-hydroxypiperidin-1-yl]-N,N-dimethyl-2,2-diphenylbutyramide hydrochloride |

All these compounds are well known approved drugs or clinical candidates for different therapeutic indications with "safe in man" trials completed.

The inventors have also tested pharmaceutically acceptable salts and hydrate forms of the above compounds and have confirmed that these also maintain the activity against the virus.

The experimental results obtained by the inventors and described in the Experimental Section below demonstrate that all these compounds are useful for the treatment of a SARS-Cov-2 infection in a subject.

Accordingly, a first object of the invention is a compound selected from the compounds above and pharmaceutically acceptable salt therof, for use in the treatment of a SARS-Cov-2 infection in a subject.

A second object of the invention is a pharmaceutical composition comprising i) a compound selected from the compounds above and pharmaceutically acceptable salts thereof, and ii) at least one inert pharmaceutically acceptable excipient, for use in the treatment of a SARS-Cov-2 infection in a subject.

A third object of the invention is a method of treating of a SARS-Cov-2 infection in a subject, comprising administering the subject a compound selected from ther compounds above and pharmaceutically acceptable salts therof.

### DETAILED DESCRIPTION OF THE INVENTION

A first object of the present invention is a compound selected from the compounds of Table 1 below and pharmaceutically acceptable salts thereof, for use in the treatment of a SARS-Cov-2 infection in a subject:

**Table 1**

| **N.** | **Generic/Drug Name** | **Chemical Name** |
|---|---|---|
| 1 | METHOTREXAT E | N-[4-[N-(2,4-Diaminopteridin-6-ylmethyl)-N-methylamino]benzoyl]-L-glutamic acid |
| 2 | Trimetrexate | 2,4-Diamino-5-methyl-6-[(3,4,5-trimethoxyanilino)methyl]quinazoline 5-Methyl-6-[[(3,4,5-trimetoxyphenyl)am ino] methyl]-2,4-quinazol inedia mine |
| 3 | Pralatrexate | N-[4-[2-(2,4-Diaminopteridin-6-yl)-1-(2-propynyl)ethyl]benzoyl]-L-glutamic acid 10-Propargyl-10-deazaaminopterin |
| 4 | CH-4051 | N-[4-[2-(2,4-Diaminoquinazolin-6-yl)ethyl]benzoyl]-4-methylene-L-glutamic acid |
| 5 | CH-1504 | N-[4-[2-(2,4-Diaminoquinazolin-6-yl)ethyl]benzoyl]-4-methylene-DL-glutamic acid |
| 6 | L-MDAM | N-[4-[2-(2,4-Diaminopteridin-6-yl)ethyl]benzoyl]-4-methylene-L-glutamic acid L-gamma-Methylene-10-deazaaminopterin |
| 7 | Talotrexin | Nalpha-(4-Amino-4-deoxypteroyl)-Ndelta-hemiphthaloyl-L-ornithine |
| 8 | Edatrexate | 10-Ethyl-10-deazaaminopterin N-[4-[1-(2,4-Diamino-6-pteridinylmethyl)propyl]benzoyl]-L-glutamic acid |
| 9 | MX-68 | N-[4-(2,4-Diaminopteridin-6-ylmethyl)-3,4-dihydro-2H-1,4-benzothiazin-7-ylcarbonyl]-L-(3'-homo)glutamic acid 2(S)-[4-(2,4-Diaminopteridin-6-ylmethyl)-3,4-dihydro-2H-1,4-benzothiazin-7-ylcarboxamido]adipic acid |
| 10 | Aminopterin | N-[4-(2,4-diamino-6-pteridinylmethylamino)benzolyl]-L-glutamic acid |
| 11 | MTX-gamma-GTP-3 | Nalpha-[4-[N-(2,4-Diamino-6-pteridinylmethyl)-N-methylamino]benzoyl]-Ndelta-[2-[2-(hexadecanoyloxy)-1,1-bis(hexadecanoyloxymethyl)ethylamino]-2-oxoethyl]-L-glutamine |
| 12 | 10-Deazaaminopt erin | N-[4-[2-(2,4-Diaminopteridin-6-yl)ethyl]benzoyl]-L-glutamic acid |
| 13 | Setanaxib | 2-(2-Chlorophenyl)-4-[3-(dimethylamino)phenyl]-5-methyl-1H-pyrazolo[4,3-c]pyridine-3,6(2H,5H)-dione |
| 14 | 640486 | 4-Methyl-2-phenyl-5-(thien-2-ylmethyl)-2,3,5,6-tetrahydro-1H-pyrazolo[4,3-c]pyridine-3,6-dione |
| 15 | 640491 | 2-(1,3-Benzothiazol-2-yl)-4-methyl-5-(2-methylbutyl)-2,3,5,6-tetrahydro-1H-pyrazolo[4,3-c]pyridine-3,6-dione |
| 16 | 640493 | 5-Benzyl-4-ethyl-2-(4-fluorophenyl)-2,3,5,6-tetrahydro-1H-pyrazolo[4,3-c]pyridine-3,6-dione |
| 17 | 640497 | 2-(1,3-Benzothiazol-2-yl)-4-ethyl-5-(pyridin-2-ylmethyl)-2,3,5,6-tetrahydro-1H-pyrazolo[4,3-c]pyridine-3,6-dione |
| 18 | 640498 | 2-(1,3-Benzothiazol-2-yl)-4-ethyl-5-(2-methoxyethyl)-2,3,5,6-tetrahydro-1H-pyrazolo[4,3-c]pyridine-3,6-dione |
| 19 | G KT-136901 | 2-(2-Chlorophenyl)-4-methyl-5-(pyridin-2-ylmethyl)-2,3,5,6-tetrahydro-1H-pyrazolo[4,3-c]pyridine-3,6-dione hydrochloride |
| 20 | PIK-III | 4'-(Cyclopropylmethyl)-N2-(pyridin-4-yl)-4,5'-bipyrimidine-2,2'-diamine |
| 21 | 781398 | 4'-[(Benzyloxy)methyl]-N2-(pyridin-4-yl)-4,5'-bipyrimidine-2,2'-diamine |
| 22 | 781406 | 1-[[4'-(Cyclopropylmethyl)-2-(pyridin-4-ylamino)-4,5'-bipyrimidin-2'-yl]amino]propan-2-ol |
| 23 | 781408 | 2-[[4'-(Cyclopropylmethyl)-2-(pyridin-4-ylamino)-4,5'-bipyrimidin-2'-yl]amino]ethanol |
| 24 | 781410 | 1-[[4'-(Cyclopropylmethyl)-2-(pyridin-4-ylamino)-4,5'-bipyrimidin-2'-yl]amino]-2-methylpropan-2-ol |
| 25 | 781424 | 4'-Methyl-N2-(pyridin-4-yl)-4,5'-bipyrimidine-2,2'-diamine |
| 26 | Arotinoid acid | 4-[2-(5,5,8,8-Tetramethyl-5,6,7,8-tetrahydronaphthalen-2-yl)-1(E)-propenyl]benzoic acid |
| 27 | Palovarotene | 4-[(E)-2-[5,5,8,8-Tetramethyl-3-(1H-pyrazol-1-ylmethyl)-5,6,7,8-tetrahydronaphthalen-2-yl]vinyl]benzoic acid |
| 28 | Ro-136298 | p-[(E)-2-(5,6,7,8-Tetrahydro-5,5,8,8-tetramethyl-2-naphthyl)propenyl]benzoic acid ethyl ester (E)-4-[2-(5,6,7,8-Tetrahydro-5,5,8,8-tetramethyl-2-naphthalenyl)-1-propenyl]benzoic acid ethyl ester |
| 29 | Etarotene | Ethyl 4-[2(E)-(5,5,8,8-Tetramethyl-5,6,7,8-tetrahydro-2-naphthyl)propenyl]phenylsulfone |
| 30 | Mofarotene | 4-[2-[4-[2(E)-(5,5,8,8-Tetramethyl-5,6,7,8-tetrahydro-2-naphthyl)-1-propenyl]phenoxy]ethyl]morpholine |
| 31 | Nelfinavir Mesilate | (3S,4aS,8aS)-N-tert-Butyl-2-[2(R)-hydroxy-3(R)-(3-hydroxy-2-methylbenzamido)-4-(phenylsulfanyl)butyl]perhydroisoquinoline-3-carboxamide methanesulfonate |
| 32 | WAY-600 | 6-(1H-Indol-5-yl)-4-(4-morpholinyl)-1-[1-(pyridin-3-ylmethyl)piperidin-4-yl]-1H-pyrazolo[3,4-d]pyrimidine |
| 33 | CFI-400945 | (1R,2S)-2-[3-[(E)-2-[4-[(cis-2,6-Dimethylmorpholin-4-yl)methyl]phenyl]vinyl]-1H-indazol-6-yl]-5'-methoxyspiro[cyclopropane-1,3'-indol]-2'(1'H)-one (2E)-but-2-enedioate |
| 34 | VE-822 (Berzosertib) | 3-[3-[4-[(Methylamino)methyl]phenyl]-1,2-oxazol-5-yl]-5-[4-(propan-2-ylsulfonyl)phenyl]pyrazin-2-amine |
| 35 | Tandutinib | N-(4-Isopropoxyphenyl)-4-[6-methoxy-7-[3-(1-piperidinyl)propoxy]quinazolin-4-yl]piperazine-1-carboxamide |
| 36 | Bemesetron | 1alphaH,3alpha,5alphaH-Tropan-3-yl-3,5-dichlorobenzoate; 3,5-Dichlorobenzoic acid endo-8-methyl-8-azabicyclo[3.2.1]oct-3-yl ester |
| 37 | Eltrombopag | 3'-[2(Z)-[1-(3,4-Dimethylphenyl)-3-methyl-5-oxo-4,5-dihydro-1H-pyrazol-4-ylidene]hydrazino]-2'-hydroxybiphenyl-3-carboxylic acid |
| 38 | Idarubicin hydrochloride | 4-Demethoxydaunorubicin hydrochloride; (1S,3S)-3-Acetyl-1,2,3,4,6,11-hexahydro-3,5,12-trihydroxy-6,11-dioxo-1-naphthacenyl 3-amino-2,3,6-trideoxy-alpha-L-lyxo-hexopyranoside hydrochloride; (7S,9S)-9-Acetyl-7-[(3-amino-2,3,6-trideoxy-alpha-L-lyxo-hexopyranosyl)oxy]-7,8,9,10-tetrahydro-6,9,11-trihydroxy-5,12-naphthacenedione hydrochloride; 4-Demethoxydaunomycin hydrochloride |
| 39 | Verteporfin | (4R,4aS)-18-Ethenyl-4,4a-dihydro-3,4-bis(methoxycarbonyl)-4a,8,14,19-tetramethyl-24H,26H-benzo[b]porphine-9,13-dipropanoic acid monomethyl ester |
| 40 | Halofantrine hydrochloride | (±)-1,3-Dichloro-9-[3-(dibutylamino)-1-hydroxypropyl]-6-(trifluoromethyl)phenanthrene hydrochloride 3-(Dibutylamino)-1-[1,3-dichloro-6-(trifluoromethyl)phenanthren-9-yl]propan-1-ol |
| 41 | Metoprine | 5-(3,4-Dichlorophenyl)-6-methylpyrimidine-2,4-diamine |
| 42 | Pyrimethamine | 5-(4-Chlorophenyl)-6-ethyl-2,4-pyrimidinediamine |
| 43 | MZPES | 5-(3-Azido-4-chlorophenyl)-6-ethyl-2,4-pyrimidine-diamine monoethanesulfonate |
| 44 | Sipatrigine | 4-Amino-2-(4-methylpiperazin-1-yl)-5-(2,3,5-trichlorophenyl)pyrimidine 2-(4-Methylpiperazin-1-yl)-5-(2,3,5-trichlorophenyl)pyrimidine-4-amine |
| 45 | BW-4030W9 2 | 5-(2,3-Dichlorophenyl)-6-(fluoromethyl)pyrimidine-2,4-diamine |
| 46 | 3'-fluorobenzylspi perone | 3-[(3-fluorophenyl)methyl]-8-[4-(4-fluorophenyl)-4-oxobutyl]-1-phenyl-1,3,8-triazaspiro[4.5]decan-4-one |
| 47 | brefeldin-a | (1R,2S,7S,13S,15S)-2,15-Dihydroxy-7-methyl-6-oxabicyclo[11.3.0]hexadeca-3(E),11(E)-dien-5-one [1S-(1R*,2E,6R*,10E,11aR*,13R*,14aS*)]-1,6,7,8,9,11a,12,13,14,14a-Decahydro-1,13-dihydroxy-6-methyl-4H-cyclopent[f]oxacyclotridecin-4-one |
| 48 | BS-181 | N5-(6-Aminohexyl)-N7-benzyl-3-isopropylpyrazolo[1,5-a]pyrimidine-5,7-diamine |
| 49 | IPAG | 2-(2-adamantyl)-1-(4-iodophenyl)guanidine |
| 50 | M asitinib (AB1010) | 4-[(4-methylpiperazin-1-yl)methyl]-N-[4-methyl-3-[(4-pyridin-3-yl-1,3-thiazol-2-yl)amino]phenyl]benzamide |
| 51 | MCOPPB | 1-[1-(1-Methylcyclooctyl)piperidin-4-yl]-2-[3(R)-piperidinyl]-1H-benzimidazole |
| 52 | MG-132 | Benzyloxycarbonyl-L-leucyl-L-leucyl-L-leucinal |
| 53 | PHA-665752 | 5-(2,6-Dichlorobenzylsulfonyl)-3(Z)-[3,5-dimethyl-4-[2(R)-(pyrrolidin-1-ylmethyl)pyrrolidin-1-ylcarbonyl]-1H-pyrrol-2-ylmethylene]-2,3-dihydro-1H-indol-2-one |
| 54 | SAR405 | (8S)-9-[(5-Chloropyridin-3-yl)methyl]-2-[(3R)-3-methylmorpholin-4-yl]-8-(trifluoromethyl)-6,7,8,9-tetrahydro-4H-pyrimido[1,2-a]pyrimidin-4-one |
| 55 | Thioguanosine | 2-amino-9-[(2R,3R,4S,5R)-3,4-dihydroxy-5-(hydroxymethyl)oxolan-2-yl]-3H-purine-6-thione |
| 56 | VU-0364739 | N-[2-[1-(3-Fluorophenyl)-4-oxo-1,3,8-triazaspiro[4.5]dec-8-yl]ethyl]naphthalen-2-carboxamide |
| 57 | ADOMEGLIVAN T | N-(4-[(1S)-1-[(4'-tert-Butyl-2,6-dimethylbiphenyl-4-yl)oxy]-4,4,4-trifluorobutyl]benzoyl)-beta-alanine |
| 58 | CYCLOHEXIMID E | 4-[2(R)-[(1S,3S,5S)-3,5-Dimethyl-2-oxocyclohexyl]-2-hydroxyethyl]piperidine-2,6-dione |
| 59 | UNC0646 | N-(1-Cyclohexylpiperidin-4-yl)-6-methoxy-7-[3-(1-piperidinyl)propoxy]-2-[4-(propan-2-yl)perhydro-1,4-diazepin-1-yl]quinazolin-4-amine |
| 60 | VPS34-IN-1 | 1-[[2-[(2-chloro-4-pyridinyl)amino]-4'-(cyclopropylmethyl)[4,5'-bipyrimidin]-2'-yl]amino]-2-methyl-2-propanol |
| 61 | N D-630 | 2-[1-[(2R)-2-(2-Methoxyphenyl)-2-(tetrahydro-2H-pyran-4-yloxy)ethyl]-5-methyl-6-(1,3-oxazol-2-yl)-2,4-dioxo-1,4-dihydrothieno[2,3-d]pyrimidin-3(2H)-yl]-2-methylpropanoic acid |
| 62 | NVP-BHG712 | 4-methyl-3-{[1-methyl-6-(3-pyridinyl)-1H-pyrazolo[3,4-d]pyrimidin-4-yl]amino}-N-[3-(trifluoromethyl)phenyl]benzamide |
| 63 | Cathepsin Inhibitor 1 | 3-tert-butyl-N-{(1S)-1-(3-chlorobenzyl)-2-[(cyanomethyl)amino]-2-oxoethyl}-1-methyl-1H-pyrazole-5-carboxamide |
| 64 | MF63 | 2-(6-Chloro-1H-phenanthro[9,10-d]imidazol-2-yl)benzene-1,3-dicarbonitrile |
| 65 | CENICRIVIROC | (-)-(S)-8-[4-(2-Butoxyethoxy)phenyl]-1-isobutyl-N-[4-(1-propyl-1H-imidazol-5-ylmethylsulfinyl)phenyl]-1,2,3,4-tetrahydro-1-benzazocine-5-carboxamide |
| 66 | TYRPHOSTIN A9 | (3,5-Di-tert-butyl-4-hydroxybenzylidene)malononitrile |
| 67 | GSK2194069 | 4-[4-(1-Benzofuran-6-yl)phenyl]-5-[1-(cyclopropylcarbonyl)pyrrolidin-3(S)-ylmethyl]-3,4-dihydro-2H-1,2,4-triazol-3-one |
| 68 | PD153035 | 4-(3-Bromophenylamino)-6,7-dimethoxyquinazoline |
| 69 | NU 7441 | 8-(Dibenzo[b,d]thien-4-yl)-2-(4-morpholinyl)-4H-1-benzopyran-4-one |
| 70 | CHIR-265 | 1-Methyl-N-[4-(trifluoromethyl)phenyl]-5-[2-[4-(trifluoromethyl)-1H-imidazol-2-yl]pyridin-4-yloxy]-1H-benzimidazol-2-amine |
| 71 | GW3965 | 2-[3-[3-[N-[2-Chloro-3-(trifluoromethyl)benzyl]-N-(2,2-diphenylethyl)amino]propoxy]phenyl]acetic acid |
| 72 | MMV665852 | 1,3-Bis(3,4-dichlorophenyl)urea |
| 73 | PB49673382 | N-(2-chlorophenyl)-2-[(2E)-2-[(2-morpholin-4-yl-4-phenyl-1,3-thiazol-5-yl)methylidene]hydrazinyl]-5-nitrobenzenesulfonamide |
| 74 | DHCaA | (1R,2S,3R)-9-(2,3-Dihydroxycyclopentyl)adenine (1R,2S,3R)-3-(6-Amino-9H-purin-9-yl)-1,2-cyclopentanediol |
| 75 | Coumarin 7 | (1R,2S,3R)-3-(6-Amino-9H-purin-9-yl)-1,2-cyclopentanediol |
| 76 | Nexinhib20 | 4,4-dimethyl-1-(3-nitrophenyl)-2-(1H-1,2,4-triazol-1-yl)-1-penten-3-one |
| 77 | Casin | 2-[(6-Phenyl-2,3,4,9-tetrahydro-1H-carbazol-1-yl)amino]ethanol |
| 78 | Bepridil | 1-[1-(N-Benzylanilino)-3-isobutoxy-2-propyl]pyrrolidine monohydrochloride monohydrate |
| 79 | Tetrandrine (Fanchinine) | (1beta)-(S,S)-6,6',7,12-Tetramethoxy-2,2'-dimethylberbaman |
| 80 | U-18666° | 3beta-[2-(Diethylamino)ethoxy]androst-5-en-17-one hydrochloride |
| 81 | CP-640186 | 1-[1'-(Anthracen-9-ylcarbonyl)-1,4'-bipiperidin-3(R)-yl]-1-(4-morpholinyl)methanone |
| 82 | CLOMIPHENE CITRATE | 2-[4-(2-Chloro-1,2-diphenylvinyl)phenoxy]-N,N-diethylethanamine citrate |
| 83 | PF-2545920 | 2-[4-[1-Methyl-4-(4-pyridyl)-1H-pyrazol-3-yl]phenoxymethyl]quinoline succinate |
| 84 | TILORONE | 2,7-Bis[2-(diethylamino)ethoxy]-9H-fluoren-9-one |
| 85 | STF-62247 | N-(3-Methylphenyl)-4-(4-pyridyl)thiazol-2-amine |
| 86 | Pyrintegrin | N-(Cyclopropylmethyl)-4-[[4-(3,4-dihydro-6-hydroxy-1(2H)-quinolinyl)-2-pyrimidinyl]amino]benzenesulfonamide |
| 87 | Y-134 | 1-[6-Hydroxy-2-(4-hydroxyphenyl)-1-benzothien-3-yl]-1-[4-(4-isopropylpiperazin-1-yl)phenyl]methanone |
| 88 | PPT | 1,3,5-Tris(4-hydroxyphenyl)-4-propyl-1H-pyrazole |
| 89 | Igmesine | (+)-N-(Cyclopropylmethyl)-N-[1-ethyl-1,4-diphenyl-3(E)-butenyl]-N-methylamine |
| 90 | Cyclobenzaprin e Hydrochloride | 3-(5H-Dibenzo[a,d]cyclohepten-5-ylidene)-N,N-dimethylpropan-1-amine hydrochloride |
| 91 | Endoxifen Hydrochloride | 4-[1-[4-[2-(Methylamino)ethoxy]phenyl]-2-phenyl-1-butenyl]phenol hydrochloride |
| 92 | GNF-5837 | 1-[2-Fluoro-5-(trifluoromethyl)phenyl]-3-[4-methyl-3-[2-oxo-3-(1H-pyrrol-2-ylmethylene)-2,3-dihydro-1H-indol-6-ylamino]phenyl]urea |
| 93 | Salinomycin | 2(R)-[6(R)-[5(R)-[(2S,5S,7R,9S,10S,12R,15R)-2-[5(R)-Ethyl-5-hydroxy-6(S)-methyltetrahydropyran-2(R)-yl]-15-hydroxy-2,10,12-trimethyl-1,6,8-trioxadispiro[4.1.5.3]pentadec-13-en-9-yl]-2(S)-hydroxy-1(S),3(S)-dimethyl-4-oxoheptyl]-5(S)-methyltetrahydropyran-2(R)-yl]butyric acid |
| 94 | Trifluoperazine dihydrochlorid e | 10-[3-(4-Methylpiperazin-1-yl)-2-(trifluoromethyl)-10H-phenothiazine dihydrochloride |
| 95 | UNC0642 | 2-(4,4-Difluoropiperidin-1-yl)-6-methoxy-N-[1-(propan-2-yl)piperidin-4-yl]-7-[3-(pyrrolidin-1-yl)propoxy]quinazolin-4-amine |
| 96 | Ciclesonide | 16alpha,17alpha-[(R)-Cyclohexylmethylenedioxy]-11beta-hydroxy-21-(isobutyryloxy)pregna-1,4-dien-3-one (R)-11beta,16alpha,17,21-Tetrahydroxypregna-1,4-diene-3,20-dione cyclic 16,17-acetal with cyclohexanecarboxaldehyde, 21-isobutyrate |
| 97 | CGP-71683 | N-[[trans-4-[[(4-Amino-2-quinazolinyl)amino]methyl]cyclohexyl]methyl]-1-naphthalenesulfonamide |
| 98 | Monensin Sodium | 4(S)-[(2S,5R,7S,8R,9S)-2-[(2S,2'R,3'S,5R,5'R)-2-Ethyl-5'-[6(R)-hydroxy-3(S),5(R)-dimethyl-6-(phenylaminocarbonyloxymethyl)tetrahydropyran-2(S)-yl]-3'-methylperhydro-2,2'-bifuran-5-yl]-9-hydroxy-2,8-dimethyl-1,6-dioxaspiro[4.5]dec-7-yl]-3(R)-methoxy-2(S)-methylpentanoic acid sodium salt |
| 99 | AG1024 | 2-(3-Bromo-5-tert-butyl-4-hydroxybenzylidene)propanedinitrile 2-(3-Bromo-5-tert-butyl-4-hydroxybenzylidene)malononitrile |
| 100 | Triclocarban | 3-(4-Chlorofenil)-1-(3,4-diclorofenil)urea |
| 101 | CP-640186 hydrochloride | 1-[1'-(Anthracen-9-ylcarbonyl)-1,4'-bipiperidin-3(R)-yl]-1-(4-morpholinyl)methanone hydrochloride |
| 102 | Bithinol | 2,2'-Thio-bis(4,6-dichlorophenol), Bis(2-hydroxy-3,5-dichlorophenyl) sulfide |
| 103 | Tyrphostin 9 | (3,5-Di-tert-butyl-4-hydroxybenzylidene)malononitrile |
| 104 | YM201636 | 6-Amino-N-[3-[4-(4-morpholinyl)pyrido[3',2':4,5]furo[3,2-d]pyrimidin-2-yl]phenyl]pyridine-3-carboxamide |
| 105 | 4E1RCat | 4-[3-[5-(4-Nitrophenyl)furan-2-ylmethylene]-2-oxo-5-phenyl-2,3-dihydro-1H-pyrrol-1-yl]benzoic acid |
| 106 | Amodiaquine hydrochloride | 4-(7-Chloroquinolin-4-ylamino)-2-(diethylaminomethyl)phenol hydrochloride |
| 107 | PD-161570 | 1-tert-Butyl-3-[6-(2,6-dichlorophenyl)-2-[4-(diethylamino)butylamino]pyrido[2,3-d]pyrimidin-7-yl]urea |
| 108 | Ixazomib | 1(R)-[2-(2,5-Dichlorobenzamido)acetamido]-3-methylbutylboronic acid |
| 109 | thiothixene | (Z)-N,N-Dimethyl-9-[3-(4-methyl-1-piperazinyl)propylidene]thioxanthene-2-sulfonamide |
| 110 | Loperamide Hydrochloride | 4-[4-(4-Chlorophenyl)-4-hydroxypiperidin-1-yl]-N,N-dimethyl-2,2-diphenylbutyramide hydrochloride |

The identification in a subject of a SARS-Cov-2 infection is usually based on the analysis of the presence of viral RNA in biological material from the subject, preferably specimens from upper and lower respeiractory tract, such as nasopharyngeal/oropharyngeal swabs, nasal aspirate, nasal wash or saliva, sputum, tracheal aspirate or bronchoalveolar lavage. The subject to be treated may have one or more symptoms of COVID-19 or be asymptomatic.

As used herein the term "pharmaceutically acceptable salts" of the compounds of Table 1 above refers to addition salts of these compounds with acids or bases that form nontoxic acid anions or cations.

Examples of acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate.

Examples of base addition salts include ammonium salts; alkali metal salts such as sodium, lithium and potassium salts; alkaline earth metal salts such as aluminum, calcium and magnesium salts; salts with organic bases such as dicyclohexylamine salts and N-methyl-D-glucamine; and salts with amino acids such as arginine, lysine, ornithine, and so forth. Also, basic nitrogen-containing groups may be quaternized with such agents as: lower alkyl halides, such as methyl, ethyl, propyl, and butyl halides; dialkyl sulfates such as dimethyl, diethyl, dibutyl; diamyl sulfates; long chain halides such as decyl, lauryl, myristyl and stearyl halides; arylalkyl halides such as benzyl bromide and others.

The salts may be formed by conventional means, such as by reacting the free form of the compound with one or more equivalents of the appropriate acid or base in a solvent or medium in which the salt is insoluble, such as for example water or ethanol, which is removed under vacuum or by freeze drying.

The compounds above may be in an anhydrous or a hydrate form.

The present invention also includes prodrugs of the compounds described above.

As used herein, the term "prodrug" refers to a biologically inactive compound which can be metabolized in the body to produce a compound of Table 1 above. An example, without limitation, of prodrugs are ester of the compounds according to the invention, that facilitate transmittal across a cell membrane where water solubility is not beneficial, but then are metabolically hydrolysed once inside the cell where water solubility is beneficial. The compound according to the first object of the invention is administered in form of a pharmaceutical composition formed by admixture of the compound with one or more pharmaceutically acceptable excipients.

Thus, a second object of the invention is a pharmaceutical composition comprising a compound selected from the compounds of Table 1 and pharmaceutically acceptable salts thereof, and at least one inert pharmaceutically acceptable excipient, for use in the treatment of a SARS-Cov-2 infection in a subject.

The route of administration of the compound or pharmaceutical composition of the present invention depends on the specific compound used or contained in the pharmaceutical composition and is in accordance with known methods for administration, which can be, for example inhalation, injection or infusion by intravenous, parenterally, topically, rectally, nasally, buccally, vaginally or via an implanted reservoir, or by sustained release systems. The term parenteral as used herein includes intravenous, intraperitoneal, intracerebral, intrathecal, intracranial, intramuscular, intraarticular, intrasynovial, intrasternal, intraocular, intraarterial, subcutaneous, and intracutaneous or intralesional injection or infusion techniques.

In one preferred embodiment, the invention provides for administration of the compound or the pharmaceutical composition of the invention such that a sufficient concentration of the compound is reached in the respiratory tract.

Preferably, this is obtained by direct administration of the compound or pharmaceutical composition of the present invention to the respiratory tract. The direct administration to the respiratory tract may be the sole route of administration of the compound or composition or may be combined with administration of the compaounsd or composition via other systemic routes.

For direct administration of the compound or composition of the invention, a wide variety of devices that are designed for the direct delivery of pharmaceutical compositions and therapeutic formulations to the respiratory tract, may be used. In one aspect of the present invention, the compound or pharmaceutical composition of the present invention is administered in aerosolized or inhaled form.

The pharmaceutical composition of the present invention for direct administration to the respiratory tract as described above, can be in form of an aerosol formulation, as a dry powder or a solution or suspension with a diluent.

Preferably, the pharmaceutically acceptable excipient includes any and all solvents, diluents, or other vehicle, dispersion or suspension aids, surface active agents, isotonic agents, thickening or emulsifying agents, preservatives, solid binders, lubricants and the like, as suited to the particular dosage form desired.

Some examples of materials which can serve as pharmaceutically acceptable excipient include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; sterilized water; Ringer's solution; buffered saline; dextrose solution; maltodextrin solution; ethyl alcohol; and phosphate buffer solutions.

Moreover, the composition of the present invention may be formulated into inhalable or injectable dosage forms such as solutions, suspensions, and emulsions by further adding diluents, dispersants, and surfactants.

Further, the composition of the present invention may be suitably formulated using appropriate methods known in the art or by the method disclosed in Remington's Pharmaceutical Science (recent edition), Mack Publishing Company, Easton Pa.

The terms "pharmaceutically acceptable" and "physiologically acceptable" are intended to define, without any particular limitation, any material suitable for preparing a pharmaceutical composition to be administered to a living being.

The dosage forms can also contain other traditional ingredients such as: preservatives, stabilizers, surfactants, buffers, and salts for regulating osmotic pressure, emulsifiers, sweeteners, colorants, flavourings and the like.

The amount of said compound in the pharmaceutical composition of the present invention is the the amount usually employed for other indications of the specific compound.

The amount can vary over a wide range depending on known factors, for example, the molecule used, the severity of the disease, the patient's body weight, the dosage form, the chosen route of administration, and the number of administrations per day. However, a person skilled in the art can determine the optimum amount in easily and routinely manner.

Also specific dosage and treatment regimens for any particular patient will depend upon a variety of factors, including for example the activity and half life of the specific compound employed, the age, body weight, general health status, sex, diet, severity and course of the disease.

The dosage forms of the pharmaceutical composition of the present invention can be prepared by techniques that are familiar to a pharmaceutical chemist, and comprise mixing, granulation, compression, dissolution, sterilization and the like.

A third object of the invention is a method of treating a SARS-Cov-2 infection in a subject, comprising administering to said subject a compound selected from the compounds of Table 1 above and pharmaceutically acceptable salts therof.

The invention will be further described in the following examples, which do not limit the scope of the invention described in the claims.

### EXPERIMENTAL PART

### 1. Computer-Aided Drug Design

The EXSCALATE platform, the most powerful computing resources currently based in Europe to empower smart in-silico drug design (co-funded by the H2020-FET-HPC ANTAREX project), was exploited to perform High Performance Computing (HPC) simulations, with the final aim to select molecules active against the SARS-CoV-2 virus.

The crystal structures of the main functional units of SARS-CoV-2 proteome were obtained from the Protein Data Bank; Table 2 reports the list of the proteins analysed, with the corresponding PDB code. Homology models of the proteins for which the crystal structure is not available were generated and used.

In particular the SARS-CoV-2 proteins most interesting as target to identify antiviral drugs, were selected.

**Table 2**

| **Proteins** | **PDB code** |
|---|---|
| 3CL protease | 6LU7 |
| N-protein | 6VYO |
| NSP3 | 6W02 |
| NSP6 | De novo model |
| NSP9 | 6W4B |
| NSP12 | 7BV2 |
| NSP13 | Homology Model |
| NSP14 | Homology Model |
| NSP15 | 6W01 |
| NSP16 | 6W4H |
| PL protease | 6W9C |
| Spike-ACE2 | 6M0J |

Molecular dynamics simulations on the SARS-CoV-2 proteins were performed to explore the conformational space of their active sites, and select several protein conformations particularly suitable for docking simulations. MD simulations were carried out on the protein structures, prepared ad hoc in order to optimize the 3D structure from a chemical and conformational point of view. The structure was firstly subjected to a cycle of energy minimization by steepest descent methods to eliminate all initial steric clashes and obtain a pre-equilibrated model to start from. Then a 100 ps restrained MD simulation (typically 1000 KJ/mole force constant) was performed on the solvent atoms to equilibrate water molecules keeping the solute restrained. Finally, a production run was performed to generate a 1microsecond trajectory with a total of 20.000 collected. Post HPC-run analysis of the results was performed.

High Performance Computing (HPC) simulation was conducted to virtual screen against the SARS-CoV-2 proteins, the Safe in Man (SIM) library, containing commercialized and under development drugs, already proved safe in man (> 10.000 drugs), and the Fraunhofer's BROAD Repurposing Library, containing 5400 marketed drugs and clinical stage compounds and molecules with known mode of action. Duplicates between the libraries were removed before ligand preparation, where all compounds were converted to 3D and prepared with Schrödinger's LigPrep tool. This process generated multiple states for stereoisomers, tautomers, ring conformations (1 stable ring conformer by default) and protonation states. In particular, another Schrödinger package, Epik, was used to assign tautomers and protonation states that would be dominant at a selected pH range (pH=7±1). Ambiguous chiral centers were enumerated, allowing a maximum of 32 isomers to be produced from each input structure. Then, an energy minimization was performed with the OPLS3 force.

The simulation was performed using LiGen^{™} (Ligand Generator), the de novo structure based virtual screening software, designed and developed to run on HPC architectures, which represent the most relevant tool of the EXSCALATE platform. LiGen^{™} is formed by a set of tools that can be combined in a user-defined manner to generate project centric workflows. In particular, LiGenDock is a docking module using LiGenScore to compute the scoring function and the LiGenPass and LiGenPocket modules to obtain the 3D structure of the binding site. Both the docking algorithm implemented in LiGen^{™}, the pharmacophoric docking (LiGenPh4) and the geometrical docking (LiGenGeodock), as well as the different scoring functions calculated, were used in this study to explore different protocols of Virtual Screening (VS) and select the best one in terms of performance.

Docking settings were validated through either redocking (where possible) or docking of known ligands. When tautomers were involved, only the one, with the best docking score, was chosen. The docking score values that predict the binding affinity of the molecules in the protein binding site, are reported (the higher, the better).

Virtual screening was performed on the main SARS-CoV-2 proteins to evaluate the potential poly-pharmacological effect of the compounds on the COVID-19 virus. A total score, corresponding to the sum of the docking scores obtained for each protein, was also calculated for each molecule and used to prioritize the most interesting molecules. Table 3 reports the results obtained for the best scored molecules, selected for further validation of the antiviral activity in in vitro experiments.

**Table 3. LiGen docking score results**

| **N.** | **Compound (Current) Name** | **3CL** | **N-prot** | **NSP3** | **NSP6** | **NSP9** | **NSP12** | **NSP13** | **NSP14** | **NSP15** | **NSP16** | **Plpro** | **Spike-ACE2** | **TotaL score** |
|---|---|---|---|---|---|---|---|---|---|---|---|---|---|---|
| 1 | METHOTREXATE | 4.00 | 4.34 | 4.32 | 4.35 | 3.50 | 4.18 | 4.47 | 5.77 | 4.53 | 5.82 | 4.48 | 5.12 | 54.88 |
| 2 | Trimetrexate | 5.04 | nd | nd | 4.26 | 4.06 | 4.82 | nd | nd | 4.39 | 5.59 | 4.54 | nd | 32.70 |
| 3 | Pralatrexate | 4.56 | 4.38 | 4.06 | 5.26 | 3.93 | 4.52 | 4.87 | 4.10 | 5.48 | 4.72 | 4.77 | 4.83 | 55.49 |
| 4 | CH-4051 | 4.73 | nd | 4.88 | 5.57 | 3.82 | 4.27 | 5.31 | nd | 4.76 | 5.14 | 4.64 | nd | 43.13 |
| 5 | CH-1504 | 4.27 | 4.83 | 5.05 | 5.11 | 3.80 | 4.47 | n.d. | nd | 5.14 | 5.63 | 5.15 | 4.63 | 48.08 |
| 6 | L-MDAM | 5.59 | nd | 4.59 | 5.32 | 3.99 | 4.40 | 4.95 | nd | 4.41 | 5.57 | 4.53 | nd | 43.35 |
| 7 | Talotrexin | 5.23 | nd | nd | 5.38 | 4.07 | 4.75 | nd | nd | 4.93 | nd | 4.71 | 5.33 | 34.39 |
| 8 | Edatrexate | 5.80 | nd | 4.90 | 5.21 | 3.58 | 4.50 | nd | nd | 4.58 | 5.68 | 4.85 | nd | 39.09 |
| 9 | MX-68 | 4.92 | 4.81 | 4.46 | 4.90 | 4.13 | 5.33 | 4.32 | nd | 5.15 | 5.57 | 4.74 | nd | 48.33 |
| 10 | Aminopterin | 4.71 | 4.29 | 3.98 | 4.53 | 3.73 | 4.20 | 4.63 | 6.01 | 4.86 | 5.45 | 4.21 | 5.59 | 56.20 |
| 12 | 10-Deazaaminopter in | 4.22 | nd | 4.97 | 4.88 | 4.43 | 4.40 | 4.75 | nd | 5.22 | 5.30 | 4.78 | 5.52 | 48.46 |
| 13 | Setanaxib | 5.10 | 4.79 | 5.10 | 5.13 | 4.77 | 5.02 | 5.16 | 5.67 | 4.49 | 4.66 | 5.44 | 5.70 | 61.04 |
| 14 | 640486 | 4.88 | 5.40 | 5.07 | 5.17 | 4.81 | 4.66 | 5.18 | 6.11 | 4.89 | 4.90 | 4.16 | 5.06 | 60.31 |
| 15 | 640491 | 5.67 | 4.70 | 4.80 | 5.42 | 4.88 | 4.72 | nd | 6.59 | 5.19 | 5.70 | 4.73 | 5.26 | 57.67 |
| 16 | 640493 | 4.80 | 4.81 | 5.70 | 5.48 | 4.54 | 4.88 | 5.06 | nd | 5.46 | 5.63 | 4.81 | nd | 51.17 |
| 17 | 640497 | 5.22 | 5.07 | nd | 5.29 | 4.67 | 4.75 | n.d. | nd | 5.23 | nd | nd | nd | 30.23 |
| 18 | 640498 | 4.60 | 4.78 | 4.61 | 4.60 | 4.04 | 4.45 | 5.08 | 6.18 | n.d. | 5.03 | 4.81 | 4.80 | 52.97 |
| 19 | GKT-136901 | 5.36 | 4.93 | 5.77 | 5.50 | 4.10 | 4.67 | nd | 6.52 | 5.47 | 5.58 | 5.14 | nd | 53.04 |
| 20 | PIK-III | 4.83 | 4.67 | 5.29 | 5.16 | 4.10 | 4.46 | 4.27 | nd | 5.78 | 5.16 | 4.82 | 4.61 | 53.16 |
| 21 | 781398 | 4.94 | 5.04 | 4.94 | 4.83 | 4.14 | 4.54 | 5.25 | nd | 5.00 | 5.09 | 5.02 | 5.02 | 53.80 |
| 22 | 781406 | 4.75 | 5.21 | 5.46 | 4.91 | 3.95 | 4.70 | 5.06 | 6.21 | 5.40 | 5.04 | 5.23 | nd | 55.91 |
| 23 | 781408 | 4.97 | 4.91 | 5.78 | 4.90 | 3.87 | 4.32 | 5.23 | nd | 5.41 | 5.24 | 4.44 | 5.05 | 54.13 |
| 24 | 781410 | 5.45 | 5.36 | 6.00 | 5.22 | 3.95 | 4.82 | 5.24 | 6.89 | 5.28 | 5.57 | 5.21 | 5.84 | 64.84 |
| 25 | 781424 | 4.83 | 4.68 | 5.28 | 5.15 | 4.31 | 4.30 | 4.85 | 5.86 | 5.14 | 5.29 | 5.03 | 4.66 | 59.36 |
| 26 | Arotinoid acid | 5.56 | 5.69 | 5.45 | 4.45 | 5.20 | 5.30 | 5.39 | 7.16 | 5.50 | 5.68 | 5.51 | 6.51 | 67.38 |
| 27 | Palovarotene | 5.56 | 5.66 | 5.56 | 4.70 | 5.28 | 5.14 | 5.04 | 6.20 | 4.65 | 6.01 | 6.21 | 6.23 | 66.24 |
| 28 | Ro-136298 | 5.49 | 4.68 | nd | 5.52 | 4.72 | 5.48 | 5.68 | nd | 5.51 | 6.74 | 5.32 | nd | 49.14 |
| 29 | Etarotene | 5.58 | nd | nd | 5.52 | 4.65 | 5.15 | nd | nd | 5.63 | nd | 5.56 | nd | 32.10 |
| 30 | Mofarotene | 5.19 | 5.78 | 5.64 | 5.50 | 5.39 | 5.36 | nd | nd | 5.34 | 5.40 | 4.90 | nd | 48.50 |
| 31 | Nelfinavir Mesilate | 5.33 | 5.34 | 4.57 | 4.64 | 4.57 | 4.91 | 4.79 | 5.11 | 5.12 | 5.61 | 5.07 | 5.66 | 60.73 |
| 32 | WAY-600 | 4.54 | 4.97 | 5.91 | 5.78 | 4.91 | 5.04 | 4.57 | 5.41 | 5.50 | 5.69 | 5.23 | 5.65 | 63.19 |
| 33 | CFI-400945 | 6.05 | 5.75 | nd | 5.58 | 4.48 | 5.48 | nd | nd | 5.85 | nd | 5.72 | nd | 38.92 |
| 34 | VE-822 (Berzosertib) | 4.27 | 5.31 | 4.95 | 4.33 | 4.09 | 5.00 | 4.71 | 5.41 | 4.80 | 6.65 | 4.90 | 6.21 | 60.63 |
| 35 | Tandutinib | 4.65 | 4.84 | 4.47 | 4.51 | 5.17 | 4.49 | 4.07 | 5.10 | 5.08 | 3.92 | 4.93 | 5.83 | 57.08 |
| 36 | Bemesetron | 5.02 | 4.74 | 5.36 | 4.79 | 3.89 | 4.47 | 4.53 | 5.83 | 4.76 | 5.06 | 5.11 | 4.67 | 58.24 |
| 37 | Eltrombopag | 5.53 | 5.29 | 5.75 | 4.78 | 4.81 | 4.89 | 4.59 | 5.44 | 4.60 | 5.13 | 5.64 | 6.05 | 62.49 |
| 38 | Idarubicin hydrochloride | 5.26 | 4.60 | 5.09 | 3.76 | 4.50 | 4.64 | 4.67 | 5.77 | 4.45 | 4.63 | 4.63 | 5.73 | 57.73 |
| 39 | Verteporfin | 4.50 | 4.26 | 4.57 | 4.25 | 4.80 | 5.39 | 4.29 | 3.41 | 5.36 | 5.83 | 4.96 | 5.73 | 57.35 |
| 40 | Halofantrine hydrochloride | 5.43 | 5.07 | 4.88 | 4.79 | 4.70 | 4.61 | 4.68 | 5.10 | 5.26 | 5.84 | 4.90 | 5.14 | 60.40 |
| 41 | Metoprine | 4.97 | 4.43 | 5.04 | 5.20 | 4.79 | 4.40 | 4.89 | 5.76 | 5.04 | 4.78 | 4.73 | 4.92 | 58.95 |
| 42 | Pyrimethamine | 4.47 | 4.67 | 4.27 | 3.92 | 4.56 | 4.42 | 4.47 | 5.88 | 5.59 | 4.64 | 4.55 | 4.38 | 55.81 |
| 43 | MZPES | 4.94 | 5.49 | 4.64 | 4.70 | 3.82 | 4.46 | 4.66 | 6.06 | 4.96 | 4.93 | 5.24 | 4.47 | 58.37 |
| 44 | Sipatrigine | 5.07 | 4.92 | 4.90 | 6.31 | 4.69 | 4.63 | 4.97 | 6.12 | 5.65 | 5.01 | 5.05 | 5.42 | 62.75 |
| 45 | BW-4030W92 | 4.80 | 4.88 | 5.10 | 4.85 | 4.33 | 4.32 | 4.90 | 5.65 | 5.27 | 5.31 | 4.82 | 4.62 | 58.84 |
| 46 | Succinobucol | 4.73 | 5.14 | 4.78 | 3.83 | 5.14 | 5.24 | 4.44 | 4.99 | 4.38 | 5.47 | 5.18 | 7.07 | 60.38 |
| 47 | Camobucol | 5.67 | 4.54 | nd | nd | 4.21 | 5.31 | 5.22 | nd | nd | 5.95 | 5.57 | nd | 36.47 |
| 48 | Elsibucol | 5.51 | nd | nd | 5.23 | nd | 5.20 | nd | nd | 5.91 | nd | 5.80 | nd | 27.65 |
| 49 | Probucol | 5.69 | 5.86 | 5.18 | 5.16 | 4.98 | 5.50 | 5.02 | 5.54 | 5.56 | 5.45 | 5.61 | 6.16 | 65.71 |
| 46 | 3'-fluorobenzylspip erone | 5.33 | 5.28 | 5.46 | 4.97 | 4.07 | 4.27 | 4.24 | 5.35 | 4.55 | 4.55 | 5.43 | 6.23 | 59.72 |
| 47 | brefeldin-a | 4.95 | 4.69 | 4.50 | 5.35 | 3.96 | 4.77 | 4.64 | 5.28 | 4.93 | 5.67 | 5.14 | 5.13 | 59.01 |
| 48 | BS-181 | 4.77 | 5.13 | 5.16 | 5.02 | 3.72 | 4.47 | 3.94 | 5.40 | 5.52 | 5.33 | 4.27 | 5.19 | 57.93 |
| 49 | IPAG | 4.65 | 5.15 | 5.00 | 4.38 | 4.53 | 5.02 | 4.76 | 6.09 | 5.07 | 5.28 | 5.18 | 5.14 | 60.24 |
| 50 | Masitinib (AB1010) | 5.85 | 5.69 | 4.91 | 5.01 | 5.04 | 5.03 | 4.46 | 6.86 | 4.85 | 5.00 | 5.02 | 6.66 | 64.37 |
| 51 | MCOPPB | 5.60 | 5.15 | 5.07 | 4.61 | 4.25 | 4.76 | 4.74 | 4.72 | 5.16 | 5.53 | 5.19 | 5.18 | 59.95 |
| 52 | MG-132 | 5.55 | 5.00 | 4.26 | 4.86 | 4.52 | 4.63 | 4.55 | 4.67 | 5.49 | 5.26 | 4.51 | 5.22 | 58.51 |
| 53 | PHA-665752 | 4.65 | 5.29 | 4.95 | 5.05 | 4.71 | 5.34 | 5.66 | 5.54 | 4.84 | 5.63 | 5.48 | 6.48 | 63.62 |
| 54 | SAR405 | 5.25 | 4.79 | 4.24 | 5.72 | 4.11 | 4.60 | 4.66 | 5.04 | 5.48 | 5.20 | 5.13 | 5.20 | 59.42 |
| 55 | thioguanosine | 4.12 | 4.27 | 4.31 | 5.59 | 4.79 | 4.27 | 4.64 | 5.16 | 4.90 | 5.25 | 4.52 | 5.10 | 56.91 |
| 56 | VU-0364739 | 5.73 | 4.46 | 5.13 | 4.70 | 4.82 | 5.05 | 5.25 | 6.78 | 4.88 | 5.40 | 5.07 | 5.83 | 63.10 |
| 57 | ADOMEGLIVANT | nd | nd | 5.27 | 5.64 | 4.14 | 5.35 | nd | nd | nd | 5.63 | 5.27 | nd | 31.30 |
| 58 | CYCLOHEXIMIDE | 4.29 | 4.38 | 4.97 | 5.02 | 4.64 | 4.56 | 4.26 | 5.54 | 5.37 | 4.84 | 5.23 | 4.45 | 57.54 |
| 59 | UNC0646 | 4.55 | 4.91 | 4.69 | 4.60 | 3.91 | 4.94 | 4.93 | 5.91 | 4.96 | 4.48 | 4.91 | 6.04 | 58.82 |
| 60 | VPS34-IN-1 | 4.88 | nd | 5.84 | 5.61 | 4.42 | 5.19 | nd | nd | 5.81 | 6.07 | 4.95 | nd | 42.78 |
| 61 | ND-630 | 5.14 | nd | 5.70 | nd | 3.83 | 5.07 | 4.84 | nd | 5.42 | nd | 4.72 | nd | 34.74 |
| 62 | NVP-BHG712 | 5.37 | 5.30 | 5.50 | 5.00 | 5.14 | 5.34 | 4.89 | 7.60 | 5.11 | 6.13 | 5.87 | 6.94 | 68.20 |
| 63 | Cathepsin Inhibitor 1 | 4.26 | 4.71 | 5.13 | 4.80 | 4.88 | 4.45 | 4.72 | 6.11 | 6.15 | 5.28 | 5.10 | 5.36 | 60.95 |
| 64 | MF63 | 5.77 | 5.88 | 6.47 | 6.39 | 5.17 | 4.99 | 5.45 | nd | 6.08 | 5.90 | 5.36 | nd | 57.45 |
| 65 | CENICRIVIROC | 4.93 | nd | nd | nd | 5.25 | 4.76 | nd | 4.86 | nd | nd | 5.75 | nd | 25.54 |
| 66 | TYRPHOSTIN A9 | 5.34 | 5.31 | 5.23 | 5.48 | 4.26 | 4.68 | 4.86 | 6.02 | 5.55 | 5.48 | 5.18 | 4.95 | 62.34 |
| 67 | GSK2194069 | 4.95 | 5.20 | 5.27 | 4.11 | 4.90 | 5.25 | 4.85 | 7.26 | 5.11 | 6.06 | 5.31 | 5.51 | 63.79 |
| 68 | PD153035 | 4.58 | 4.84 | 5.22 | 5.35 | 3.87 | 4.71 | 5.15 | 6.26 | 4.77 | 4.76 | 5.03 | 4.51 | 59.04 |
| 69 | NU 7441 | 5.73 | 5.49 | 5.42 | 4.54 | 4.27 | 4.99 | 4.46 | 6.69 | 5.64 | 5.50 | 5.45 | 5.36 | 63.54 |
| 70 | CHIR-265 | 5.96 | 5.23 | 4.87 | 4.25 | 4.73 | 5.06 | 4.41 | 7.17 | 5.38 | 6.39 | 5.29 | 5.58 | 64.32 |
| 71 | GW3965 | 4.68 | 5.00 | 4.44 | 4.63 | 4.23 | 4.26 | 4.63 | 5.00 | 5.43 | 5.38 | 4.90 | 4.83 | 57.41 |
| 72 | MMV665852 | 5.16 | 5.40 | nd | 5.60 | 4.68 | 4.97 | 5.13 | 6.10 | 5.29 | 5.68 | 5.08 | nd | 53.09 |
| 73 | PB49673382 | 5.61 | 6.05 | 5.70 | 5.71 | 4.69 | 5.23 | nd | nd | 5.88 | 5.88 | 5.76 | nd | 50.50 |
| 74 | DHCaA | 4.48 | 4.80 | 5.04 | 4.74 | 4.09 | 4.53 | 4.46 | 5.83 | 4.79 | 5.08 | 4.88 | 5.46 | 58.19 |
| 75 | Coumarin 7 | 4.97 | 5.22 | 5.62 | 4.85 | 4.36 | 4.79 | nd | nd | 4.98 | nd | 5.82 | nd | 40.61 |
| 76 | Nexinhib20 | 5.20 | 5.00 | nd | 5.13 | 3.95 | 4.66 | 4.98 | nd | 5.31 | 5.73 | 4.73 | 4.79 | 49.48 |
| 77 | casin | 4.90 | 4.46 | 5.17 | 4.46 | 4.61 | 4.26 | 4.38 | 4.94 | 4.40 | 5.75 | 4.49 | 4.85 | 56.68 |
| 78 | bepridil | 4.58 | 4.45 | 5.68 | 4.44 | 3.93 | 4.12 | 4.18 | 5.84 | 5.01 | 4.50 | 4.79 | 5.10 | 56.62 |
| 79 | Tetrandrine (Fanchinine) | 4.97 | 4.80 | 4.88 | 5.87 | 4.95 | 5.22 | 4.16 | 3.78 | 5.96 | 4.68 | 4.70 | 5.95 | 59.92 |
| 80 | U-18666A | 4.68 | 4.99 | 5.59 | 4.16 | 4.48 | 4.90 | 4.54 | 4.72 | 4.39 | 4.96 | 5.68 | 5.23 | 58.31 |
| 81 | CP-640186 | 5.33 | 4.97 | 4.81 | 4.56 | 4.59 | 5.04 | 4.79 | 5.46 | 4.25 | 5.56 | 5.67 | 5.73 | 60.76 |
| 82 | CLOMIPHENE | 5.74 | 5.06 | 5.69 | 4.84 | 5.07 | 4.59 | 4.52 | 6.83 | 4.55 | 5.61 | 5.46 | 6.32 | 64.27 |
| 83 | PF-2545920 | 5.64 | 5.00 | 5.34 | 4.98 | 5.36 | 4.66 | 4.70 | 6.57 | 4.86 | 5.59 | 5.27 | 4.99 | 62.96 |
| 84 | TILORONE | 4.67 | 4.24 | 3.84 | 4.22 | 3.79 | 4.49 | 3.54 | 4.05 | 4.14 | 4.08 | 4.35 | 5.46 | 50.86 |
| 85 | STF-62247 | 4.69 | 5.13 | 5.42 | 5.29 | 4.05 | 4.53 | 4.92 | 6.07 | 5.49 | 5.83 | 5.47 | 5.72 | 62.61 |
| 86 | pyrintegrin | 5.27 | 4.62 | 4.63 | 4.56 | 4.58 | 4.63 | 5.26 | 6.76 | 4.74 | 5.44 | 4.94 | 5.75 | 61.17 |
| 87 | Y-134 | 5.39 | 5.47 | 5.28 | 5.52 | 5.62 | 5.27 | 5.04 | 6.37 | 5.30 | 5.94 | 5.90 | 6.90 | 68.01 |
| 88 | PTT | 4.93 | 5.11 | 5.99 | 4.88 | 4.35 | 5.17 | 5.01 | 7.10 | 5.04 | 4.73 | 5.81 | 5.46 | 63.59 |
| 89 | Igmesine | 4.49 | 4.74 | 5.84 | 5.61 | 4.65 | 4.66 | 4.09 | 6.40 | 5.85 | 4.13 | 4.71 | 4.82 | 59.98 |
| 90 | Cyclobenzaprine Hydrochloride | 4.51 | 4.66 | 5.87 | 4.88 | 4.37 | 4.40 | 4.87 | 5.63 | 4.90 | 4.94 | 5.35 | 4.44 | 58.82 |
| 91 | Endoxifen Hydrochloride | 4.90 | 4.96 | 6.03 | 5.36 | 4.31 | 4.80 | 4.46 | 5.71 | 5.61 | 5.27 | 4.72 | 5.12 | 61.25 |
| 92 | GNF-5837 | 6.25 | 6.34 | 5.19 | 4.23 | 4.50 | 5.13 | 4.59 | 5.21 | 5.33 | 5.99 | 5.25 | 6.45 | 64.45 |
| 93 | Salinomycin | 4.74 | 5.34 | 4.70 | 3.99 | 5.23 | 5.19 | 4.48 | 3.95 | 4.19 | 4.84 | 5.83 | 6.91 | 59.40 |
| 94 | Trifluoperazine dihydrochloride | 4.64 | 4.52 | 4.53 | 5.66 | 4.19 | 4.12 | 3.90 | 4.37 | 5.43 | 4.62 | 4.29 | 5.77 | 56.05 |
| 95 | UNC0642 | 5.25 | 4.60 | 5.27 | 4.82 | 4.21 | 4.88 | 5.04 | 4.97 | 4.49 | 5.74 | 5.11 | 5.76 | 60.14 |
| 96 | Ciclesonide | 4.63 | 5.11 | 4.63 | 5.95 | 5.00 | 4.64 | 4.73 | 7.21 | 5.21 | 4.52 | 5.69 | 5.57 | 62.88 |
| 97 | CGP-71683 | 6.04 | 4.79 | 5.22 | 4.33 | 4.63 | 5.04 | 4.27 | 4.94 | 5.20 | 5.70 | 5.19 | 5.00 | 60.34 |
| 98 | Monensin Sodium | 4.81 | 5.95 | 4.46 | 4.39 | 4.92 | 4.86 | 4.89 | 5.95 | 5.55 | 4.12 | 5.36 | 7.03 | 62.30 |
| 99 | AG1024 | 4.92 | 5.06 | 5.85 | 5.17 | 4.13 | 4.56 | 4.97 | 5.80 | 5.38 | 5.38 | 4.74 | 5.05 | 61.01 |
| 100 | Triclocarban | 5.06 | 5.53 | 5.24 | 5.29 | 4.62 | 5.04 | 5.11 | 5.82 | 5.53 | 5.96 | 4.89 | 5.53 | 63.63 |
| 101 | CP-640186 hydrochloride | 5.33 | 4.97 | 4.81 | 4.56 | 4.59 | 5.04 | 4.79 | 5.46 | 4.25 | 5.56 | 5.67 | 5.73 | 60.76 |
| 102 | Bithionol | 4.86 | 4.74 | 5.00 | 4.81 | 4.43 | 4.74 | 4.22 | 5.38 | 5.61 | 5.83 | 4.61 | 4.58 | 58.82 |
| 103 | Tyrphostin 9 | 5.34 | 5.31 | 5.23 | 5.48 | 4.26 | 4.68 | 4.86 | 6.02 | 5.55 | 5.48 | 5.18 | 4.95 | 62.34 |
| 104 | YM201636 | 5.55 | 5.49 | 5.28 | 5.31 | 5.17 | 5.05 | 4.73 | 7.53 | 5.38 | 4.90 | 5.57 | 6.27 | 66.23 |
| 105 | 4E1RCat | 5.61 | 5.37 | 5.60 | 4.97 | 5.28 | 5.07 | 5.64 | 7.36 | 5.03 | 5.79 | 5.19 | 6.83 | 67.74 |
| 106 | Amodiaquine hydrochloride | 4.82 | 4.83 | 4.91 | 4.74 | 4.92 | 4.65 | 4.95 | 6.11 | 5.08 | 4.70 | 5.39 | 5.89 | 61.01 |
| 107 | PD-161570 | 5.18 | 5.51 | 5.41 | 5.40 | 5.37 | 5.29 | 4.30 | 5.21 | 4.76 | 4.50 | 4.78 | 5.70 | 61.41 |
| 108 | Ixazomib | 4.54 | 4.41 | 4.68 | 4.75 | 4.57 | 4.45 | 4.80 | 5.80 | 5.68 | 4.30 | 4.82 | 5.29 | 0.00 |
| 109 | thiothixene | 4.02 | 5.40 | 4.07 | 4.33 | 4.40 | 4.09 | 4.46 | 5.82 | 5.68 | 4.57 | 4.59 | 5.47 | 56.91 |
| 110 | Loperamide Hydrochloride | 4.66 | 4.93 | 6.11 | 4.29 | 5.00 | 4.87 | 5.06 | 6.89 | 4.84 | 5.17 | 5.24 | 5.09 | 62.15 |

### 2. In vitro screening of activity against SARS-CoV-2

The compounds selected as active by the CADD analysis were tested in a SARS-CoV 2 VeroE6-EGFP HTS antiviral Assay. This assay is based on the monitoring of cytopathic effects induced by viral infection of VeroE6 cells constitutively expressing an enhanced green fluorescent protein (EGFP).

In details, the VeroE6-EGFP reporter cell line was received from JNJ whereas the SARS-CoV-2 used for infections is a Belgian strain (BetaCov/Belgium/GHB-03021/2020) present in the KU Leuven laboratories.

The test compounds were dissolved at 10mM in dimethylsulphoxide (DMSO) and then diluted in cell culture medium to the required final concentrations. DMSO final concentration was below 0.5%. Then, compounds were mixed with with 0.01 MOI of the virus 8000 VeroE6-EGFP cells/well in 384-well plates. Cells infected by Sars-CoV 2 and treated with Remdesivir 20uM were used as positive control (inhibition 100%) whereas cells infected by Sars-CoV 2 without Remdesivir as negative control (0% inhibition).

After incubation at 37°C for 5 days the EGFP signal of each well was recorded using an argon laser-scanning microscope.

Standard whole-well fluorescence plate readout was performed (self-optimizing protocol, ∼6min per 384-well plate, 4 reads/well) and the fluorescence total intensity was measured for both test compounds and control wells.

High-content imaging readout was also performed using a 5x objective, allowing to capture almost the entire well of a 384-well plate at once (auto-focus on each well, no binning, 1 channel). Two values were obtained from high content imaging:
% Confluence: indicates the increase in confluence of VEROE6-EGFP cells in the test compound wells compared to the control wells. This parameter is based on the quantification of the total surface of the field that gives a green fluorescent signal, due to the presence of EGPF-positive cells in Sars-Cov2 infected wells treated with test compounds compared to untreated control wells: higher values mean that there are a large number of cells on the microtiter plate bottom surface, small values mean that most of the fluorescence is lost (i.e. the cells died). In the table attached, we repoted the Maximum confluence reached (%) after test compound treatment of Sars-Cov2 infected cells.
IC50 (µM): indicates the half maximal concentration able to recover the cytopatic effect of infection and is a measure of the potency of a substance in inhibiting viral included cell death. It was determined from dose -response curves obtained testing the compounds antiviral effect at 8 different concentrations.

All the test compounds are able to inhibit virus replication and consequent cytopathic effects of the virus compared to the control, in particular, they recover cell growth at least to 30% of cell confluence. Interestingly, 50 of the 83 selected compounds (60%) are able to recover more than 50% of cell confluence.

The results obtained for the compounds tested are shown in Table 4 below.

**Table 4**

| **N.** | **Compound (current) Name** | **IC50 (µM)** | **Maximum confluence reached (%)** |
|---|---|---|---|
| 1 | METHOTREXATE | 0.05 | 64 |
| 2 | Trimetrexate | 0.33 | 78.23 |
| 3 | Pralatrexate | 0.01 | 76.65 |
| 10 | Aminopterin | 0.35 | 72.54 |
| 13 | Setanaxib | 7.57 | 101.52 |
| 20 | PIK-III | 7.92 | 98.17 |
| 26 | Arotinoid acid | 33.71 | 105.73 |
| 31 | Nelfinavir Mesilate | 7.08 | 59.49 |
| 32 | WAY-600 | 9.43 | 67.69 |
| 33 | CFI-400945 | 11.91 | 105.46 |
| 34 | VE-822 (Berzosertib) | 2.67 | 99.21 |
| 35 | Tandutinib | 4.23 | 80.09 |
| 36 | bemesetron | 43.9 | 69.05 |
| 37 | Eltrombopag | 58.08 | 63.17 |
| 38 | Idarubicin hydrochloride | 58.94 | 92.62 |
| 39 | verteporfin | 30 | 89.5 |
| 40 | Halofantrine hydrochloride | 5.31 | 51.82 |
| 41 | Metoprine | 2.34 | 58.02 |
| 46 | 3'-fluorobenzylspiperone | 7.66 | 43.04 |
| 47 | brefeldin-a | 0.02 | 121.63 |
| 48 | BS-181 | 12.93 | 47.31 |
| 49 | IPAG | 12.76 | 81.85 |
| 50 | Masitinib (AB1010) | 11.9 | 57.11 |
| 51 | MCOPPB | 13.14 | 57.77 |
| 52 | MG-132 | 0.32 | 82.81 |
| 53 | PHA-665752 | 4.97 | 95.69 |
| 54 | SAR405 | 10 | 89 |
| 55 | thioguanosine | 15.59 | 51.54 |
| 56 | VU-0364739 | 11.64 | 98.46 |
| 57 | ADOMEGLIVANT | 10.55 | 39.53 |
| 58 | CYCLOHEXIMIDE | 1.16 | 63.7 |
| 59 | UNC0646 | 12.03 | 80.08 |
| 60 | VPS34-IN-1 | 0.4 | 45.62 |
| 61 | ND-630 | 0.38 | 78.18 |
| 62 | NVP-BHG712 | 5.26 | 88.43 |
| 63 | Cathepsin Inhibitor 1 | 8.98 | 96.18 |
| 64 | MF63 | 13.29 | 98.48 |
| 65 | CENICRIVIROC | 55 | 70.92 |
| 66 | TYRPHOSTIN A9 | 0.83 | 47.6 |
| 67 | GSK2194069 | 16.98 | 31.06 |
| 68 | PD153035 | 13.37 | 54.45 |
| 69 | NU 7441 | 10 | 90.45 |
| 70 | CHIR-265 | 14.64 | 44.1 |
| 71 | GW3965 | 27.71 | 38.01 |
| 72 | MMV665852 | 3.97 | 45.87 |
| 73 | PB49673382 | 6.42 | 53.9 |
| 74 | DHCaA | 3.69 | 37.98 |
| 75 | Coumarin 7 | 0.36 | 33.14 |
| 76 | Nexinhib20 | 4.33 | 50.44 |
| 77 | casin | 14.15 | 50.81 |
| 78 | bepridil | 3.86 | 30.41 |
| 79 | Tetrandrine (Fanchinine) | 11.66 | 99.64 |
| 80 | U-18666A | 1.15 | 44.66 |
| 81 | CP-640186 | 3.78 | 51 |
| 82 | CLOMIPHENE CITRATE | 13.62 | 54.73 |
| 83 | PF-2545920 | 25.26 | 36.73 |
| 84 | TILORONE | 16.44 | 68.89 |
| 85 | STF-62247 | 4.11 | 47.81 |
| 86 | pyrintegrin | 1.31 | 55.04 |
| 87 | Y-134 | 29.87 | 41.04 |
| 88 | PPT | 8.3 | 48.2 |
| 89 | Igmesine | 11.2 | 58.34 |
| 90 | Cyclobenzaprine Hydrochloride | 12.1 | 70,19 |
| 91 | Endoxifen Hydrochloride | 6.34 | 35.68 |
| 92 | GNF-5837 | 5.23 | 75.5 |
| 93 | Salinomycin | 0.38 | 34.36 |
| 94 | Trifluoperazine dihydrochloride | 16.65 | 37.97 |
| 95 | UNC0642 | 16.73 | 31.06 |
| 96 | Ciclesonide | 46.95 | 35.2 |
| 97 | CGP-71683 | 7.36 | 39.25 |
| 98 | Monensin Sodium | 3.18 | 38.24 |
| 99 | AG1024 | 7.23 | 37.27 |
| 100 | Triclocarban | 8.33 | 47.16 |
| 101 | CP-640186 hydrochloride | 3.78 | 50.88 |
| 102 | Bithionol | 19.1 | 31.25 |
| 103 | Tyrphostin 9 | 204.65 | 51.51 |
| 104 | YM201636 | 4.73 | 35.48 |
| 105 | 4E1RCat | 236.34 | 52.15 |
| 106 | Amodiaquine hydrochloride | 21.51 | 37.5 |
| 107 | PD-161570 | 144.52 | 34.11 |
| 108 | Ixazomib | 135.08 | 30.79 |
| 109 | thiothixene | 18.24 | 36.55 |
| 110 | Loperamide Hydrochloride | 17.16 | 34.41 |

## Claims

1. A compound selected from the compounds of Table 1
**Table 1**
| **N.** | **Generic/Drug Name** | **Chemical Name** |
|---|---|---|
| 1 | METHOTREXATE | N-[4-[N-(2,4-Diaminopteridin-6-ylmethyl)-N-methylamino]benzoyl]-L-glutamic acid |
| 2 | Trimetrexate | 2,4-Diamino-5-methyl-6-[(3,4,5-trimethoxyanilino)methyl]quinazoline 5-Methyl-6-[[(3,4,5-trimetoxyphenyl)amino]methyl]-2,4-quinazolinediamine |
| 3 | Pralatrexate | N-[4-[2-(2,4-Diaminopteridin-6-yl)-1-(2-propynyl)ethyl]benzoyl]-L-glutamic acid 10-Propargyl-10-deazaaminopterin |
| 4 | CH-4051 | N-[4-[2-(2,4-Diaminoquinazolin-6-yl)ethyl]benzoyl]-4-methylene-L-glutamic acid |
| 5 | CH-1504 | N-[4-[2-(2,4-Diaminoquinazolin-6-yl)ethyl]benzoyl]-4-methylene-DL-glutamic acid |
| 6 | L-MDAM | N-[4-[2-(2,4-Diaminopteridin-6-yl)ethyl]benzoyl]-4-methylene-L-glutamic acid L-gamma-Methylene-10-deazaaminopterin |
| 7 | Talotrexin | Nalpha-(4-Amino-4-deoxypteroyl)-Ndelta-hemiphthaloyl-L-ornithine |
| 8 | Edatrexate | 10-Ethyl-10-deazaaminopterin N-[4-[1-(2,4-Diamino-6-pteridinylmethyl)propyl]benzoyl]-L-glutamicacid |
| 9 | MX-68 | N-[4-(2,4-Diaminopteridin-6-ylmethyl)-3,4-dihydro-2H-1,4-benzothiazin-7-ylcarbonyl]-L-(3'-homo)glutamic acid 2(S)-[4-(2,4-Diaminopteridin-6-ylmethyl)-3,4-dihydro-2H-1,4-benzothiazin-7-ylcarboxamido]adipic acid |
| 10 | Aminopterin | N-[4-(2,4-diamino-6-pteridinylmethylamino)benzolyl]-L-glutamic acid |
| 11 | MTX-gamma-GTP-3 | Nalpha-[4-[N-(2,4-Diamino-6-pteridinylmethyl)-N-methylamino]benzoyl]-Ndelta-[2-[2-(hexadecanoyloxy)-1,1-bis(hexadecanoyloxymethyl)ethylamino]-2-oxoethyl]-L-glutamine |
| 12 | 10-Deazaaminopte rin | N-[4-[2-(2,4-Diaminopteridin-6-yl)ethyl]benzoyl]-L-glutamic acid |
| 13 | Setanaxib | 2-(2-Chlorophenyl)-4-[3-(dimethylamino)phenyl]-5-methyl-1H-pyrazolo[4,3-c]pyridine-3,6(2H,5H)-dione |
| 14 | 640486 | 4-Methyl-2-phenyl-5-(thien-2-ylmethyl)-2,3,5,6-tetrahydro-1H-pyrazolo[4,3-c]pyridine-3,6-dione |
| 15 | 640491 | 2-(1,3-Benzothiazol-2-yl)-4-methyl-5-(2-methylbutyl)-2,3,5,6-tetrahydro-1H-pyrazolo[4,3-c]pyridine-3,6-dione |
| 16 | 640493 | 5-Benzyl-4-ethyl-2-(4-fluorophenyl)-2,3,5,6-tetrahydro-1H-pyrazolo[4,3-c]pyridine-3,6-dione |
| 17 | 640497 | 2-(1,3-Benzothiazol-2-yl)-4-ethyl-5-(pyridin-2-ylmethyl)-2,3,5,6-tetrahydro-1H-pyrazolo[4,3-c]pyridine-3,6-dione |
| 18 | 640498 | 2-(1,3-Benzothiazol-2-yl)-4-ethyl-5-(2-methoxyethyl)-2,3,5,6-tetrahydro-1H-pyrazolo[4,3-c]pyridine-3,6-dione |
| 19 | G KT-136901 | 2-(2-Chlorophenyl)-4-methyl-5-(pyridin-2-ylmethyl)-2,3,5,6-tetrahydro-1H-pyrazolo[4,3-c]pyridine-3,6-dione hydrochloride |
| 20 | PIK-III | 4'-(Cyclopropylmethyl)-N2-(pyridin-4-yl)-4,5'-bipyrimidine-2,2'-diamine |
| 21 | 781398 | 4'-[(Benzyloxy)methyl]-N2-(pyridin-4-yl)-4,5'-bipyrimidine-2,2'-diamine |
| 22 | 781406 | 1-[[4'-(Cyclopropylmethyl)-2-(pyridin-4-ylamino)-4,5'-bipyrimidin-2'-yl]amino]propan-2-ol |
| 23 | 781408 | 2-[[4'-(Cyclopropylmethyl)-2-(pyridin-4-ylamino)-4,5'-bipyrimidin-2'-yl]amino]ethanol |
| 24 | 781410 | 1-[[4'-(Cyclopropylmethyl)-2-(pyridin-4-ylamino)-4,5'-bipyrimidin-2'-yl]amino]-2-methylpropan-2-ol |
| 25 | 781424 | 4'-Methyl-N2-(pyridin-4-yl)-4,5'-bipyrimidine-2,2'-diamine |
| 26 | Arotinoid acid | 4-[2-(5,5,8,8-Tetramethyl-5,6,7,8-tetrahydronaphthalen-2-yl)-1(E)-propenyl]benzoic acid |
| 27 | Palovarotene | 4-[(E)-2-[5,5,8,8-Tetramethyl-3-(1H-pyrazol-1-ylmethyl)-5,6,7,8-tetrahydronaphthalen-2 -yl]vi nyl] benzoic acid |
| 28 | Ro-136298 | p-[(E)-2-(5,6,7,8-Tetrahydro-5,5,8,8-tetramethyl-2-naphthyl)propenyl]benzoic acid ethyl ester (E)-4-[2-(5,6,7,8-Tetrahydro-5,5,8,8-tetramethyl-2-naphthalenyl)-1-propenyl]benzoic acid ethyl ester |
| 29 | Etarotene | Ethyl 4-[2(E)-(5,5,8,8-Tetramethyl-5,6,7,8-tetrahydro- 2-naphthyl)propenyl]phenylsulfone |
| 30 | Mofarotene | 4-[2-[4-[2(E)-(5,5,8,8-Tetramethyl-5,6,7,8-tetrahydro-2-naphthyl)-1-propenyl]phenoxy]ethyl]morpholine |
| 31 | Nelfinavir Mesilate | (3S,4aS,8aS)-N-tert-Butyl-2-[2(R)-hydroxy-3(R)-(3-hydroxy-2-methylbenzamido)-4-(phenylsulfanyl)butyl]perhydroisoquinoline-3-carboxamide methanesulfonate |
| 32 | WAY-600 | 6-(1H-Indol-5-yl)-4-(4-morpholinyl)-1-[1-(pyridin-3-ylmethyl)piperidin-4-yl]-1H-pyrazolo[3,4-d]pyrimidine |
| 33 | CFI-400945 | (1R,2S)-2-[3-[(E)-2-[4-[(cis-2,6-Dimethylmorpholin-4-yl)methyl]phenyl]vinyl]-1H-indazol-6-yl]-5'-methoxyspiro[cyclopropane-1,3'-indol]-2'(1'H)-one (2E)-but-2-enedioate |
| 34 | VE-822 (Berzosertib) | 3-[3-[4-[(Methylamino)methyl]phenyl]-1,2-oxazol-5-yl]-5-[4-(propan-2-ylsulfonyl)phenyl]pyrazin-2-amine |
| 35 | Tandutinib | N-(4-Isopropoxyphenyl)-4-[6-methoxy-7-[3-(1-piperidinyl)propoxy]quinazolin-4-yl]piperazine-1-carboxamide |
| 36 | Bemesetron | 1alphaH,3alpha,5alphaH-Tropan-3-yl-3,5-dichlorobenzoate; 3,5-Dichlorobenzoic acid endo-8-methyl-8-azabicyclo[3.2.1]oct-3-yl ester |
| 37 | Eltrombopag | 3'-[2(Z)-[1-(3,4-Dimethylphenyl)-3-methyl-5-oxo-4,5-dihydro-1H-pyrazol-4-ylidene]hydrazino]-2'-hydroxybiphenyl-3-carboxylic acid |
| 38 | Idarubicin hydrochloride | 4-Demethoxydaunorubicin hydrochloride; (1S,3S)-3-Acetyl-1,2,3,4,6,11-hexahydro-3 ,5, 12-trihydroxy-6,11-dioxo-1-naphthacenyl 3-am i no-2,3 ,6-trideoxy-alpha-L-lyxo-hexopyranoside hydrochloride; (7S,9S)-9-Acetyl-7-[(3-amino-2,3,6-trideoxy-alpha-L-lyxo-hexopyranosyl)oxy]-7,8,9,10-tetrahydro-6,9,11-trihydroxy-5,12-naphthacenedione hydrochloride; 4-Demethoxydaunomycin hydrochloride |
| 39 | Verteporfin | (4R,4aS)-18-Ethenyl-4,4a-dihydro-3,4-bis(methoxycarbonyl)-4a,8,14,19-tetramethyl-24H,26H-benzo[b]porphine-9,13-dipropanoic acid monomethyl ester |
| 40 | Halofantrine hydrochloride | (±)-1,3-Dichloro-9-[3-(dibutylamino)-1-hydroxypropyl]-6-(trifluoromethyl)phenanthrene hydrochloride 3-(Dibutylamino)-1-[1,3-dichloro-6-(trifluoromethyl)phenanthren-9-yl]propan-1-ol |
| 41 | Metoprine | 5-(3,4-Dichlorophenyl)-6-methylpyrimidine-2,4-diamine |
| 42 | Pyrimethamine | 5-(4-Chlorophenyl)-6-ethyl-2,4-pyrimidinediamine |
| 43 | MZPES | 5-(3-Azido-4-chlorophenyl)-6-ethyl-2,4-pyrimidine-diamine monoethanesulfonate |
| 44 | Sipatrigine | 4-Amino-2-(4-methylpiperazin-1-yl)-5-(2,3,5-trichlorophenyl)pyrimidine 2-(4-Methylpiperazin-1-yl)-5-(2,3,5-trichlorophenyl)pyrimidine-4-amine |
| 45 | BW-4030W92 | 5-(2,3-Dichlorophenyl)-6-(fluoromethyl)pyrimidine-2,4-diamine |
| 46 | 3'-fluorobenzylspi perone | 3-[(3-fluorophenyl)methyl]-8-[4-(4-fluorophenyl)-4-oxobutyl]-1-phenyl-1,3,8-triazaspiro[4.5]decan-4-one |
| 47 | brefeldin-a | (1R,2S,7S,13S,15S)-2,15-Dihydroxy-7-methy1-6-oxabicyclo[11.3.0]hexadeca-3(E),11(E)-dien-5-one [1S-(1R*,2E,6R*,10E,11aR*,13R*,14aS*)]-1,6,7,8,9,11a,12,13,14,14a-Decahydro-1,13-dihydroxy-6-methyl-4H-cyclopent[f]oxacyclotridecin-4-one |
| 48 | BS-181 | N5-(6-Aminohexyl)-N7-benzyl-3-isopropylpyrazolo[l,5-a]pyrimidine-5,7-diamine |
| 49 | IPAG | 2-(2-adamantyl)-1-(4-iodophenyl)guanidine |
| 50 | Masitinib (AB1010) | 4-[(4-methylpiperazin-1-yl)methyl]-N-[4-methyl-3-[(4-pyridin-3-yl-1,3-thiazol-2-yl)amino]phenyl]benzamide |
| 51 | MCOPPB | 1-[1-(1-Methylcyclooctyl)piperidin-4-yl]-2-[3(R)-piperidinyl]-1H-benzimidazole |
| 52 | MG-132 | Benzyloxycarbonyl-L-leucyl-L-leucyl-L-leucinal |
| 53 | PHA-665752 | 5-(2,6-Dichlorobenzylsulfonyl)-3(Z)-[3,5-dimethyl-4-[2(R)-(pyrrolidin-1-ylmethyl)pyrrolidin-1-ylcarbonyl]-1H-pyrrol-2-ylmethylene]-2,3-dihydro-1H-indol-2-one |
| 54 | SAR405 | (8S)-9-[(5-Chloropyridin-3-yl)methyl]-2-[(3R)-3-methylmorpholin-4-yl]-8-(trifluoromethyl)-6,7,8,9-tetrahydro-4H-pyrimido[1,2-a]pyrimidin-4-one |
| 55 | Thioguanosine | 2-amino-9-[(2R,3R,4S,5R)-3,4-dihydroxy-5-(hydroxymethyl)oxolan-2-yl]-3H-purine-6-thione |
| 56 | VU-0364739 | N-[2-[1-(3-Fluorophenyl)-4-oxo-1,3,8-triazaspiro[4.5]dec-8-yl]ethyl]naphthalen-2-carboxamide |
| 57 | ADOMEGLIVAN T | N-(4-[(1S)-1-[(4'-tert-Butyl-2,6-dimethylbiphenyl-4-yl)oxy]-4,4,4-trifluorobutyl]benzoyl)-beta-alanine |
| 58 | CYCLOHEXIMID E | 4-[2(R)-[(1S,3S,5S)-3,5-Dimethyl-2-oxocyclohexyl]-2-hydroxyethyl] piperidine-2,6-dione |
| 59 | UNC0646 | N-(1-Cyclohexylpiperidin-4-yl)-6-methoxy-7-[3-(1-piperidinyl)propoxy]-2-[4-(propan-2-yl)perhydro-1,4-diazepin-1-yl]quinazolin-4-amine |
| 60 | VPS34-IN-1 | 1-[[2-[(2-chloro-4-pyridinyl)amino]-4'-(cyclopropylmethyl)[4,5'-bipyrimidin]-2'-yl]amino]-2-methyl-2-propanol |
| 61 | N D-630 | 2-[1-[(2R)-2-(2-Methoxyphenyl)-2-(tetrahydro-2H-pyran-4-yloxy)ethyl]-5-methyl-6-(1,3-oxazol-2-yl)-2,4-dioxo-1,4-dihydrothieno[2,3-d]pyrimidin-3(2H)-yl]-2-methylpropanoic acid |
| 62 | NVP-BHG712 | 4-methyl-3-{[1-methyl-6-(3-pyridinyl)-1H-pyrazolo[3,4-d]pyrimidin-4-yl]amino}-N-[3-(trifluoromethyl)phenyl]benzamide |
| 63 | Cathepsin Inhibitor 1 | 3-tert-butyl-N-{(15)-1-(3-chlorobenzyl)-2-[(cyanomethyl)amino]-2-oxoethyl}-1-methyl-1H-pyrazole-5-carboxamide |
| 64 | MF63 | 2-(6-Chloro-1H-phenanthro[9,10-d]imidazol-2-yl)benzene-1,3-dicarbonitrile |
| 65 | CENICRIVIROC | (-)-(S)-8-[4-(2-Butoxyethoxy)phenyl]-1-isobutyl-N-[4-(1-propyl-1H-imidazol-5-ylmethylsulfinyl)phenyl]-1,2,3,4-tetrahydro-1-benzazocine-5-carboxamide |
| 66 | TYRPHOSTIN A9 | (3,5-Di-tert-butyl-4-hydroxybenzylidene)malononitrile |
| 67 | GSK2194069 | 4-[4-(1-Benzofuran-6-yl)phenyl]-5-[1-(cyclopropylcarbonyl)pyrrolidin-3(S)-ylmethyl]-3,4-dihydro-2H-1,2,4-triazol-3-one |
| 68 | PD153035 | 4-(3-Bromophenylamino)-6,7-dimethoxyquinazoline |
| 69 | NU 7441 | 8-(Dibenzo[b,d]thien-4-yl)-2-(4-morpholinyl)-4H-1-benzopyran-4-one |
| 70 | CHIR-265 | 1-Methyl-N-[4-(trifluoromethyl)phenyl]-5-[2-[4-(trifluoromethyl)-1H-imidazol-2-yl]pyridin-4-yloxy]-1H-benzimidazol-2-amine |
| 71 | GW3965 | 2-[3-[3-[N-[2-Chloro-3-(trifluoromethyl)benzyl]-N-(2,2-diphenylethyl)amino]propoxy]phenyl]acetic acid |
| 72 | MMV665852 | 1,3-Bis(3,4-dichlorophenyl)urea |
| 73 | PB49673382 | N-(2-chlorophenyl)-2-[(2E)-2-[(2-morpholin-4-yl-4-phenyl-1,3-thiazol-5-yl)methylidene]hydrazinyl]-5-nitrobenzenesulfonamide |
| 74 | DHCaA | (1R,2S,3R)-9-(2,3-Dihydroxycyclopentyl)adenine (1R,2S,3R)-3-(6-Amino-9H-purin-9-yl)-1,2-cyclopentanediol |
| 75 | Coumarin 7 | (1R,2S,3R)-3-(6-Amino-9H-purin-9-yl)-1,2-cyclopentanediol |
| 76 | Nexinhib20 | 4,4-dimethyl-1-(3-nitrophenyl)-2-(1H-1,2,4-triazol-1-yl)-1-penten-3-one |
| 77 | Casin | 2-[(6-Phenyl-2,3,4,9-tetrahydro-1H-carbazol-1-yl)amino]ethanol |
| 78 | Bepridil | 1-[1-(N-Benzylanilino)-3-isobutoxy-2-propyl]pyrrolidine monohydrochloride monohydrate |
| 79 | Tetrandrine (Fanchinine) | (1beta)-(S,S)-6,6',7,12-Tetramethoxy-2,2'-dimethylberbaman |
| 80 | U-18666° | 3beta-[2-(Diethylamino)ethoxy]androst-5-en-17-one hydrochloride |
| 81 | CP-640186 | 1-[1'-(Anthracen-9-ylcarbonyl)-1,4'-bipiperidin-3(R)-yl]-1-(4-morpholinyl)methanone |
| 82 | CLOMIPHENE CITRATE | 2-[4-(2-Chloro-1,2-diphenylvinyl)phenoxy]-N,N-diethylethanamine citrate |
| 83 | PF-2545920 | 2-[4-[1-Methyl-4-(4-pyridyl)-1H-pyrazol-3-yl]phenoxymethyl]quinoline succinate |
| 84 | TILORONE | 2,7-Bis[2-(diethylamino)ethoxy]-9H-fluoren-9-one |
| 85 | STF-62247 | N-(3-Methylphenyl)-4-(4-pyridyl)thiazol-2-amine |
| 86 | Pyrintegrin | N-(Cyclopropylmethyl)-4-[[4-(3,4-dihydro-6-hydroxy-1(2H)-quinolinyl)-2-pyrimidinyl]amino]benzenesulfonamide |
| 87 | Y-134 | 1-[6-Hydroxy-2-(4-hydroxyphenyl)-1-benzothien-3-yl]-1-[4-(4-isopropylpiperazin-1-yl)phenyl]methanone |
| 88 | PPT | 1,3,5-Tris(4-hydroxyphenyl)-4-propyl-1H-pyrazole |
| 89 | Igmesine | (+)-N-(Cyclopropylmethyl)-N-[1-ethyl-1,4-diphenyl-3(E)-butenyl]-N-methylamine |
| 90 | Cyclobenzaprine Hydrochloride | 3-(5H-Dibenzo[a,d]cyclohepten-5-ylidene)-N,N-dimethylpropan-1-amine hydrochloride |
| 91 | Endoxifen Hydrochloride | 4-[1-[4-[2-(Methylamino)ethoxy]phenyl]-2-phenyl-1-butenyl]phenol hydrochloride |
| 92 | GNF-5837 | 1-[2-FIuoro-5-(trifluoromethyl)phenyl]-3-[4-methyl-3-[2-oxo-3-(1H-pyrrol-2-ylmethylene)-2,3-dihydro-1H-indol-6-ylamino]phenyl]urea |
| 93 | Salinomycin | 2(R)-[6(R)-[5(R)-[(2S,5S,7R,9S,10S,12R,15R)-2-[5(R)-Ethyl-5-hydroxy-6(S)-methyltetrahydropyran-2(R)-yl]-15-hydroxy-2,10,12-trimethyl-1,6,8-trioxadispiro[4.1.5.3]pentadec-13-en-9-yl]-2(S)-hydroxy-1(S),3(S)-dimethyl-4-oxoheptyl]-5(S)-methyltetrahydropyran-2(R)-yl]butyric acid |
| 94 | Trifl uoperazine dihydrochloride | 10-[3-(4-Methylpiperazin-1-yl)propyl]-2-(trifluoromethyl)-10H-phenothiazine dihydrochloride |
| 95 | UNC0642 | 2-(4,4-Difluoropiperidin-1-yl)-6-methoxy-N-[1-(propan-2-yl)piperidin-4-yl]-7-[3-(pyrrolidin-1-yl)propoxy]quinazolin-4-amine |
| 96 | Ciclesonide | 16alpha,17alpha-[(R)-Cyclohexylmethylenedioxy]-11beta-hydroxy-21-(isobutyryloxy)pregna-1,4-dien-3-one (R)-11beta,16alpha,17,21-Tetrahydroxypregna-1,4-diene-3,20-dione cyclic 16,17-acetal with cyclohexanecarboxaldehyde, 21-isobutyrate |
| 97 | CGP-71683 | N-[[trans-4-[[(4-Amino-2-quinazolinyl)amino]methyl]cyclohexyl]methyl]-1-naphthalenesulfonamide |
| 98 | Monensin Sodium | 4(S)-[(2S,5R,7S,8R,9S)-2-[(2S,2'R,3'S,5R,5'R)-2-Ethyl-5'-[6(R)-hydroxy-3(S),5(R)-dimethyl-6-(phenylaminocarbonyloxymethyl)tetrahydropyran-2(S)-yl]-3'-methylperhydro-2,2'-bifuran-5-yl]-9-hydroxy-2,8-dimethyl-1,6-dioxaspiro[4.5]dec-7-yl]-3(R)-methoxy-2(S)-methylpentanoic acid sodium salt |
| 99 | AG1024 | 2-(3-Bromo-5-tert-butyl-4-hydroxybenzylidene)propanedinitrile 2-(3-Bromo-5-tert-butyl-4-hydroxybenzylidene)malononitrile |
| 100 | Triclocarban | 3-(4-Chlorofenil)-1-(3,4-diclorofenil)urea |
| 101 | CP-640186 hydrochloride | 1-[1'-(Anthracen-9-ylcarbonyl)-1,4'-bipiperidin-3(R)-yl]-1-(4-morpholinyl)methanone hydrochloride |
| 102 | Bithionol | 2,2'-Thio-bis(4,6-dichlorophenol), Bis(2-hydroxy-3,5-dichlorophenyl) sulfide |
| 103 | Tyrphostin 9 | (3,5-Di-tert-butyl-4-hydroxybenzylidene)malononitrile |
| 104 | YM201636 | 6-Amino-N-[3-[4-(4-morpholinyl)pyrido[3',2':4,5]furo[3,2-d]pyrimidin-2-yl]phenyl]pyridine-3-carboxamide |
| 105 | 4E1RCat | 4-[3-[5-(4-Nitrophenyl)furan-2-ylmethylene]-2-oxo-5-phenyl-2,3-dihydro-1H-pyrrol-1-yl]benzoic acid |
| 106 | Amodiaquine hydrochloride | 4-(7-Chloroquinolin-4-ylamino)-2-(diethylaminomethyl)phenol hydrochloride |
| 107 | PD-161570 | 1-tert-Butyl-3-[6-(2,6-dichlorophenyl)-2-[4-(diethylamino)butylamino]pyrido[2,3-d]pyrimidin-7-yl]urea |
| 108 | Ixazomib | 1(R)-[2-(2,5-Dichlorobenzamido)acetamido]-3-methylbutylboronic acid |
| 109 | thiothixene | (Z)-N,N-Dimethyl-9-[3-(4-methyl-1-piperazinyl)propylidene]thioxanthene-2-sulfonamide |
| 110 | Loperamide Hydrochloride | 4-[4-(4-Chlorophenyl)-4-hydroxypiperidin-1-yl]-N,N-dimethyl-2,2-diphenylbutyramide hydrochloride |
and pharmaceutically acceptable salts thereof, for use in the treatment of a SARS-Cov-2 infection in a subject:

2. A pharmaceutical composition comprising:
i) A compound selected from the compounds of Table 1 below
**Table 1**
| N. | Generic/Drug Name | Chemical Name |
|---|---|---|
| 1 | METHOTREXATE | N-[4-[N-(2,4-Diaminopteridin-6-ylmethyl)-N-methylamino]benzoyl]-L-glutamic acid |
| 2 | Trimetrexate | 2,4-Diamino-5-methyl-6-[(3,4,5-trimethoxyanilino)methyl]quinazoline 5-Methyl-6-[[(3,4,5-trimetoxyphenyl)amino]methyl]-2,4-quinazolinediamine |
| 3 | Pralatrexate | N-[4-[2-(2,4-Diaminopteridin-6-yl)-1-(2-propynyl)ethyl]benzoyl]-L-glutamic acid 10-Propargyl-10-deazaaminopterin |
| 4 | CH-4051 | N-[4-[2-(2,4-Diaminoquinazolin-6-yl)ethyl]benzoyl]-4-methylene-L-glutamic acid |
| 5 | CH-1504 | N-[4-[2-(2,4-Diaminoquinazolin-6-yl)ethyl]benzoyl]-4-methylene-DL-glutamic acid |
| 6 | L-MDAM | N-[4-[2-(2,4-Diaminopteridin-6-yl)ethyl]benzoyl]-4-methylene-L-glutamic acid L-gamma-Methylene-10-deazaaminopterin |
| 7 | Talotrexin | Nalpha-(4-Amino-4-deoxypteroyl)-Ndelta-hemiphthaloyl-L-ornithine |
| 8 | Edatrexate | 10-Ethyl-10-deazaaminopterin N-[4-[1-(2,4-Diamino-6-pteridinylmethyl)propyl] benzoyl]-L -glutamic acid |
| 9 | MX-68 | N-[4-(2,4-Diaminopteridin-6-ylmethyl)-3,4-dihydro-2H-1,4-benzothiazin-7-ylcarbonyl]-L-(3'-homo)glutamic acid 2(S)-[4-(2,4-Diaminopteridin-6-ylmethyl)-3,4-dihydro-2H-1,4-benzothiazin-7-ylcarboxamido]adipic acid |
| 10 | Aminopterin | N-[4-(2,4-diamino-6-pteridinylmethylamino)benzolyl]-L-glutamic acid |
| 11 | MTX-gamma-GTP-3 | Nalpha-[4-[N-(2,4-Diamino-6-pteridinylmethyl)-N-methylamino]benzoyl]-Ndelta-[2-[2-(hexadecanoyloxy)-1,1-bis(hexadecanoyloxymethyl)ethylamino]-2-oxoethyl]-L-glutamine |
| 12 | 10-Deazaaminopterin | N-[4-[2-(2,4-Diaminopteridin-6-yl)ethyl]benzoyl]-L-glutamic acid |
| 13 | Setanaxib | 2-(2-Chlorophenyl)-4-[3-(dimethylamino)phenyl]-5-methyl-1H-pyrazolo[4,3-c]pyridine-3,6(2H,5H)-dione |
| 14 | 640486 | 4-M ethyl-2-phenyl-5-(thien-2-ylmethyl)-2,3,5,6-tetrahydro-1H-pyrazolo[4,3-c]pyridine-3,6-dione |
| 15 | 640491 | 2-(1,3-Benzothiazol-2-yl)-4-methyl-5-(2-methylbutyl)-2,3,5,6-tetrahydro-1H-pyrazolo[4,3-c]pyridine-3,6-dione |
| 16 | 640493 | 5-Benzyl-4-ethyl-2-(4-fluorophenyl)-2,3,5,6-tetrahydro-1H-pyrazolo[4,3-c]pyridine-3,6-dione |
| 17 | 640497 | 2-(1,3-Benzothiazol-2-yl)-4-ethyl-5-(pyridin-2-ylmethyl)-2,3,5,6-tetrahydro-1H-pyrazolo[4,3-c]pyridine-3,6-dione |
| 18 | 640498 | 2-(1,3-Benzothiazol-2-yl)-4-ethyl-5-(2-methoxyethyl)-2,3,5,6-tetrahydro-1H-pyrazolo[4,3-c]pyridine-3,6-dione |
| 19 | GKT-136901 | 2-(2-Chlorophenyl)-4-methyl-5-(pyridin-2-ylmethyl)-2,3,5,6-tetrahydro-1H-pyrazolo[4,3-c] pyridi ne-3,6-dione hydrochloride |
| 20 | PIK-III | 4'-(Cyclopropylmethyl)-N2-(pyridin-4-yl)-4,5'-bipyrimidine-2,2'-diamine |
| 21 | 781398 | 4'-[(Benzyloxy)methyl]-N2-(pyridin-4-yl)-4,5'-bipyrimidine-2,2'-diamine |
| 22 | 781406 | 1-[[4'-(Cyclopropylmethyl)-2-(pyridin-4-ylamino)-4,5'-bipyrimidin-2'-yl]amino]propan-2-ol |
| 23 | 781408 | 2-[[4'-(Cyclopropylmethyl)-2-(pyridin-4-ylamino)-4,5'-bipyrimidin-2'-yl]amino]ethanol |
| 24 | 781410 | 1-[[4'-(Cyclopropylmethyl)-2-(pyridin-4-ylamino)-4,5'-bipyrimidin-2'-yl]amino]-2-methylpropan-2-ol |
| 25 | 781424 | 4'-Methyl-N2-(pyridin-4-yl)-4,5'-bipyrimidine-2,2'-diamine |
| 26 | Arotinoid acid | 4-[2-(5,5,8,8-Tetramethyl-5,6,7,8-tetrahydronaphthalen-2-yl)-1(E)-propenyl]benzoic acid |
| 27 | Palovarotene | 4-[(E)-2-[5,5,8,8-Tetramethyl-3-(1H-pyrazol-1-ylmethyl)-5,6,7,8-tetrahydronaphthalen-2 -yl]vinyl] benzoic acid |
| 28 | Ro-136298 | p-[(E)-2-(5,6,7,8-Tetrahydro-5,5,8,8-tetramethyl-2-naphthyl)propenyl]benzoic acid ethyl ester (E)-4-[2-(5,6,7,8-Tetrahydro-5,5,8,8-tetramethyl-2-naphthalenyl)-1-propenyl]benzoic acid ethyl ester |
| 29 | Etarotene | Ethyl 4-[2(E)-(5,5,8,8-Tetramethyl-5,6,7,8-tetrahydro-2-naphthyl)propenyl]phenylsulfone |
| 30 | Mofarotene | 4-[2-[4-[2(E)-(5,5,8,8-Tetramethyl-5,6,7,8-tetrahydro-2-naphthyl)-1-propenyl]phenoxy]ethyl]morpholine |
| 31 | Nelfinavir Mesilate | (3S,4aS,8aS)-N-tert-Butyl-2-[2(R)-hydroxy-3(R)-(3-hydroxy-2-methylbenzamido)-4-(phenylsulfanyl)butyl]perhydroisoquinoline-3-carboxamide methanesulfonate |
| 32 | WAY-600 | 6-(1H-Indol-5-yl)-4-(4-morpholinyl)-1-[1-(pyridin-3-ylmethyl)piperidin-4-yl]-1H-pyrazolo[3,4-d]pyrimidine |
| 33 | CFI-400945 | (1R,25)-2-[3-[(E)-2-[4-[(cis-2,6-Dimethylmorpholin-4-yl)methyl]phenyl]vinyl]-1H-indazol-6-yl]-5'-methoxyspiro[cyclopropane-1,3'-indol]-2'(1'H)-one (2E)-but-2-enedioate |
| 34 | VE-822 (Berzosertib) | 3-[3-[4-[(Methylamino)methyl]phenyl]-1,2-oxazol-5-yl]-5-[4-(propan-2-ylsulfonyl)phenyl]pyrazin-2-amine |
| 35 | Tandutinib | N-(4-Isopropoxyphenyl)-4-[6-methoxy-7-[3-(1-piperidinyl)propoxy]quinazolin-4-yl]piperazine-1-carboxamide |
| 36 | Bemesetron | 1alphaH,3alpha,5alphaH-Tropan-3-yl-3,5-dichlorobenzoate; 3,5-Dichlorobenzoic acid endo-8-methyl-8-azabicyclo[3.2.1]oct-3-yl ester |
| 37 | Eltrombopag | 3'-[2(Z)-[1-(3,4-Dimethylphenyl)-3-methyl-5-oxo-4,5-dihydro-1H-pyrazol-4-ylidene]hydrazino]-2'-hydroxybiphenyl-3-carboxylic acid |
| 38 | Idarubicin hydrochloride | 4-Demethoxydaunorubicin hydrochloride; (1S,3S)-3-Acetyl-1,2,3,4,6,11-hexahydro-3,5,12-trihydroxy-6,11-dioxo-1-naphthacenyl 3-amino-2,3,6-trideoxy-alpha-L-lyxo-hexopyranoside hydrochloride; (7S,9S)-9-Acetyl-7-[(3-amino-2,3,6-trideoxy-alpha-L-lyxo-hexopyranosyl)oxy]-7,8,9,10-tetrahydro-6,9,11-trihydroxy-5,12-naphthacenedione hydrochloride; 4-Demethoxydaunomycin hydrochloride |
| 39 | Verteporfin | (4R,4aS)-18-Ethenyl-4,4a-dihydro-3,4-bis(methoxycarbonyl)-4a,8,14,19-tetramethyl-24H,26H-benzo[b]porphine-9,13-dipropanoic acid monomethyl ester |
| 40 | Halofantrine hydrochloride | (±)-1,3-Dichloro-9-[3-(dibutylamino)-1-hydroxypropyl]-6-(trifluoromethyl)phenanthrene hydrochloride 3-(Dibutylamino)-1-[1,3-dichloro-6-(trifluoromethyl)phenanthren-9-yl]propan-1-ol |
| 41 | Metoprine | 5-(3,4-Dichlorophenyl)-6-methylpyrimidine-2,4-diamine |
| 42 | Pyrimethamine | 5-(4-Chlorophenyl)-6-ethyl-2,4-pyrimidinediamine |
| 43 | MZPES | 5-(3-Azido-4-chlorophenyl)-6-ethyl-2,4-pyrimidine-diamine monoethanesulfonate |
| 44 | Sipatrigine | 4-Amino-2-(4-methylpiperazin-1-yl)-5-(2,3,5-trichlorophenyl)pyrimidine 2-(4-Methylpiperazin-1-yl)-5-(2,3,5-trichlorophenyl)pyrimidine-4-amine |
| 45 | BW-4030W92 | 5-(2,3-Dichlorophenyl)-6-(fluoromethyl)pyrimidine-2,4-diamine |
| 46 | 3'-fluorobenzylspiperone | 3-[(3-fluorophenyl)methyl]-8-[4-(4-fluorophenyl)-4-oxobutyl]-1-phenyl-1,3,8-triazaspiro[4.5]decan-4-one |
| 47 | brefeldin-a | (1R,2S,7S,13S,15S)-2,15-Dihydroxy-7-methyl-6-oxabicyclo[11.3.0]hexadeca-3(E),11(E)-dien-5-one [1S-(1R*,2E,6R*,10E,11aR*,13R*,14aS*)]-1,6,7,8,9,11a,12,13,14,14a-Decahydro-1,13-dihydroxy-6-methyl-4H-cyclopent[f]oxacyclotridecin-4-one |
| 48 | BS-181 | N5-(6-Aminohexyl)-N7-benzyl-3-isopropylpyrazolo[1,5-a]pyrimidine-5,7-diamine |
| 49 | IPAG | 2-(2-adamantyl)-1-(4-iodophenyl)guanidine |
| 50 | Masitinib (AB1010) | 4-[(4-methylpiperazin-1-yl)methyl]-N-[4-methyl-3-[(4-pyridin-3-yl-1,3-thiazol-2-yl)amino]phenyl]benzamide |
| 51 | MCOPPB | 1-[1-(1-Methylcyclooctyl)piperidin-4-yl]-2-[3(R)-piperidinyl]-1H-benzimidazole |
| 52 | MG-132 | Benzyloxycarbonyl-L-leucyl-L-leucyl-L-leucinal |
| 53 | PHA-665752 | 5-(2,6-Dichlorobenzylsulfonyl)-3(Z)-[3,5-dimethyl-4-[2(R)-(pyrrolidin-1-ylmethyl)pyrrolidin-1-ylcarbonyl]-1H-pyrrol-2-ylmethylene]-2,3-dihydro-1H-indol-2-one |
| 54 | SAR405 | (8S)-9-[(5-Chloropyridin-3-yl)methyl]-2-[(3R)-3-methylmorpholin-4-yl]-8-(trifluoromethyl)-6,7,8,9-tetrahydro-4H-pyrimido[1,2-a]pyrimidin-4-one |
| 55 | Thioguanosine | 2-amino-9-[(2R,3R,4S,5R)-3,4-dihydroxy-5-(hydroxymethyl)oxolan-2-yl]-3H-purine-6-thione |
| 56 | VU-0364739 | N-[2-[1-(3-Fluorophenyl)-4-oxo-1,3,8-triazaspiro[4.5]dec-8-yl]ethyl]naphthalen-2-carboxamide |
| 57 | ADOMEGLIVANT | N-(4-[(1S)-1-[(4'-tert-Butyl-2,6-dimethylbiphenyl-4-yl)oxy]-4,4,4-trifluorobutyl]benzoyl)-beta-alanine |
| 58 | CYCLOHEXIMIDE | 4-[2(R)-[(1S,3S,5S)-3,5-Dimethyl-2-oxocyclohexyl]-2-hydroxyethyl] piperidine-2,6-dione |
| 59 | UNC0646 | N-(1-Cyclohexylpiperidin-4-yl)-6-methoxy-7-[3-(1-piperidinyl)propoxy]-2-[4-(propan-2-yl)perhydro-1,4-diazepin-1-yl]quinazolin-4-amine |
| 60 | VPS34-IN-1 | 1-[[2-[(2-chloro-4-pyridinyl)amino]-4'-(cyclopropylmethyl)[4,5'-bipyrimidin]-2'-yl]amino]-2-methyl-2-propanol |
| 61 | N D-630 | 2-[1-[(2R)-2-(2-Methoxyphenyl)-2-(tetrahydro-2H-pyran-4-yloxy)ethyl]-5-methyl-6-(1,3-oxazol-2-yl)-2,4-dioxo-1,4-dihydrothieno[2,3-d]pyrimidin-3(2H)-yl]-2-methylpropanoic acid |
| 62 | NVP-BHG712 | 4-methyl-3-{[1-methyl-6-(3-pyridinyl)-1H-pyrazolo[3,4-d]pyrimidin-4-yl]amino}-N-[3-(trifluoromethyl)phenyl]benzamide |
| 63 | Cathepsin Inhibitor 1 | 3-tert-butyl-N-{(1S)-1-(3-chlorobenzyl)-2-[(cyanomethyl)amino]-2-oxoethyl}-1-methyl-1H-pyrazole-5-carboxamide |
| 64 | MF63 | 2-(6-Chloro-1H-phenanthro[9,10-d]imidazol-2-yl)benzene-1,3-dicarbonitrile |
| 65 | CENICRIVIROC | (-)-(S)-8-[4-(2-Butoxyethoxy)phenyl]-1-isobutyl-N-[4-(1-propyl-1H-imidazol-5-ylmethylsulfinyl)phenyl]-1,2,3,4-tetrahydro-1-benzazocine-5-carboxamide |
| 66 | TYRPHOSTIN A9 | (3,5-Di-tert-butyl-4-hydroxybenzylidene)malononitrile |
| 67 | GSK2194069 | 4-[4-(1-Benzofuran-6-yl)phenyl]-5-[1-(cyclopropylcarbonyl)pyrrolidin-3(S)-ylmethyl]-3,4-dihydro-2H-1,2,4-triazol-3-one |
| 68 | PD153035 | 4-(3-Bromophenylamino)-6,7-dimethoxyquinazoline |
| 69 | NU 7441 | 8-(Dibenzo[b,d]thien-4-yl)-2-(4-morpholinyl)-4H-1-benzopyran-4-one |
| 70 | CHIR-265 | 1-Methyl-N-[4-(trifluoromethyl)phenyl]-5-[2-[4-(trifluoromethyl)-1H-imidazol-2-yl]pyridin-4-yloxy]-1H-benzimidazol-2-amine |
| 71 | GW3965 | 2-[3-[3-[N-[2-Chloro-3-(trifluoromethyl)benzyl]-N-(2,2-diphenylethyl)amino]propoxy]phenyl]acetic acid |
| 72 | MMV665852 | 1,3-Bis(3,4-dichlorophenyl)urea |
| 73 | PB49673382 | N-(2-chlorophenyl)-2-[(2E)-2-[(2-morpholin-4-yl-4-phenyl-1,3-thiazol-5-yl)methylidene]hydrazinyl]-5-nitrobenzenesulfonamide |
| 74 | DHCaA | (1R,2S,3R)-9-(2,3-Dihydroxycyclopentyl)adenine (1R,2S,3R)-3-(6-Amino-9H-purin-9-yl)-1,2-cyclopentanediol |
| 75 | Coumarin 7 | (1R,25,3R)-3-(6-Amino-9H-purin-9-yl)-1,2-cyclopentanediol |
| 76 | Nexinhib20 | 4,4-dimethyl-1-(3-nitrophenyl)-2-(1H-1,2,4-triazol-1-yl)-1-penten-3-one |
| 77 | Casin | 2-[(6-Phenyl-2,3,4,9-tetrahydro-1H-carbazol-1-yl)amino]ethanol |
| 78 | Bepridil | 1-[1-(N-Benzylanilino)-3-isobutoxy-2-propyl]pyrrolidine monohydrochloride monohydrate |
| 79 | Tetrandrine (Fanchinine) | (1beta)-(S,S)-6,6',7,12-Tetramethoxy-2,2'-dimethylberbaman |
| 80 | U-18666° | 3beta-[2-(Diethylamino)ethoxy]androst-5-en-17-one hydrochloride |
| 81 | CP-640186 | 1-[1'-(Anthracen-9-ylcarbonyl)-1,4'-bipiperidin-3(R)-yl]-1-(4-morpholinyl)methanone |
| 82 | CLOMIPHENE CITRATE | 2-[4-(2-Chloro-1,2-diphenylvinyl)phenoxy]-N,N-diethylethanamine citrate |
| 83 | PF-2545920 | 2-[4-[1-Methyl-4-(4-pyridyl)-1H-pyrazol-3-yl]phenoxymethyl]quinoline succinate |
| 84 | TILORONE | 2,7-Bis[2-(diethylamino)ethoxy]-9H-fluoren-9-one |
| 85 | STF-62247 | N-(3-Methylphenyl)-4-(4-pyridyl)thiazol-2-amine |
| 86 | Pyrintegrin | N-(Cyclopropylmethyl)-4-[[4-(3,4-dihydro-6-hydroxy-1(2H)-quinolinyl)-2-pyrimidinyl]amino]benzenesulfonamide |
| 87 | Y-134 | 1-[6-Hydroxy-2-(4-hydroxyphenyl)-1-benzothien-3-yl]-1-[4-(4-isopropylpiperazin-1-yl)phenyl]methanone |
| 88 | PPT | 1,3,5-Tris(4-hydroxyphenyl)-4-propyl-1H-pyrazole |
| 89 | Igmesine | (+)-N-(Cyclopropylmethyl)-N-[1-ethyl-1,4-diphenyl-3(E)-butenyl]-N-methylamine |
| 90 | Cyclobenzaprine Hydrochloride | 3-(5H-Dibenzo[a,d]cyclohepten-5-ylidene)-N,N-dimethylpropan-1-amine hydrochloride |
| 91 | Endoxifen Hydroch loride | 4-[1-[4-[2-(Methylamino)ethoxy]phenyl]-2-phenyl-1-butenyl]phenol hydrochloride |
| 92 | GNF-5837 | 1-[2-Fluoro-5-(trifluoromethyl)phenyl]-3-[4-methyl-3-[2-oxo-3-(1H-pyrrol-2-ylmethylene)-2,3-dihydro-1H-indol-6-ylamino]phenyl]urea |
| 93 | Salinomycin | 2(R)-[6(R)-[5(R)-[(2S,5S,7R,9S,10S,12R,15R)-2-[5(R)-Ethyl-5-hydroxy-6(S)-methyltetrahydropyran-2(R)-yl]-15-hydroxy-2,10,12-trimethyl-1,6,8-trioxadispiro[4.1.5.3]pentadec-13-en-9-yl]-2(S)-hydroxy-1(S),3(S)-dimethyl-4-oxoheptyl]-5(S)-methyltetrahydropyran-2(R)-yl]butyric acid |
| 94 | Trifluoperazi ne dihydrochloride | 10-[3-(4-Methylpiperazin-1-yl)propyl]-2-(trifluoromethyl)-10H-phenothiazine dihydrochloride |
| 95 | UNC0642 | 2-(4,4-Difluoropiperidin-1-yl)-6-methoxy-N-[1-(propan-2-yl)piperidin-4-yl]-7-[3-(pyrrolidin-1-yl)propoxy]quinazolin-4-amine |
| 96 | Ciclesonide | 16alpha,17alpha-[(R)-Cyclohexylmethylenedioxy]-11beta-hydroxy-21-(isobutyryloxy)pregna-1,4-dien-3-one (R)-11beta,16alpha,17,21-Tetrahydroxypregna-1,4-diene-3,20-dione cyclic 16,17-acetal with cyclohexanecarboxaldehyde, 21-isobutyrate |
| 97 | CGP-71683 | N-[[trans-4-[[(4-Amino-2-quinazolinyl)amino]methyl]cyclohexyl]methyl]-1-naphthalenesulfonamide |
| 98 | Monensin Sodium | 4(S)-[(2S,5R,7S,8R,9S)-2-[(2S,2'R,3'S,5R,5'R)-2-Ethyl-5'-[6(R)-hydroxy-3(S),5(R)-dimethyl-6-(phenylaminocarbonyloxymethyl)tetrahydropyran-2(S)-yl]-3'-methylperhydro-2,2'-bifuran-5-yl]-9-hydroxy-2,8-dimethyl-1,6-dioxaspiro[4.5]dec-7-yl]-3(R)-methoxy-2(S)-methylpentanoic acid sodium salt |
| 99 | AG1024 | 2-(3-Bromo-5-tert-butyl-4-hydroxybenzylidene)propanedinitrile 2-(3-Bromo-5-tert-butyl-4-hydroxybenzylidene)malononitrile |
| 100 | Triclocarban | 3-(4-Chlorofenil)-1-(3,4-diclorofenil)urea |
| 101 | CP-640186 hydrochloride | 1-[1'-(Anthracen-9-ylcarbonyl)-1,4'-bipiperidin-3(R)-yl]-1-(4-morpholinyl)methanone hydrochloride |
| 102 | Bithionol | 2,2'-Thio-bis(4,6-dichlorophenol), Bis(2-hydroxy-3,5-dichlorophenyl) sulfide |
| 103 | Tyrphostin 9 | (3,5-Di-tert-butyl-4-hydroxybenzylidene)malononitrile |
| 104 | YM201636 | 6-Amino-N-[3-[4-(4-morpholinyl)pyrido[3',2':4,5]furo[3,2-d]pyrimidin-2-yl]phenyl]pyridine-3-carboxamide |
| 105 | 4E1RCat | 4-[3-[5-(4-Nitrophenyl)furan-2-ylmethylene]-2-oxo-5-phenyl-2,3-dihydro-1H-pyrrol-1-yl]benzoic acid |
| 106 | Amodiaquine hydrochloride | 4-(7-Chloroquinolin-4-ylamino)-2-(diethylaminomethyl)phenol hydrochloride |
| 107 | PD-161570 | 1-tert-Butyl-3-[6-(2,6-dichlorophenyl)-2-[4-(diethylamino)butylamino]pyrido[2,3-d]pyrimidin-7-yl]urea |
| 108 | Ixazomib | 1(R)-[2-(2,5-Dichlorobenzamido)acetamido]-3-methylbutylboronic acid |
| 109 | thiothixene | (Z)-N,N-Dimethyl-9-[3-(4-methyl-1-piperazinyl)propylidene]thioxanthene-2-sulfonamide |
| 110 | Loperamide Hydrochloride | 4-[4-(4-Chlorophenyl)-4-hydroxypiperidin-1-yl]-N,N-dimethyl-2,2-diphenylbutyramide hydrochloride |
and pharmaceutically acceptable salts thereof, and
ii) at least one inert pharmaceutically acceptable excipient,
for use in the treatment of a SARS-Cov-2 infection in a subject

3. A compound for use according to claim 1 or a pharmaceutical composition for use according to claim 2, wherein said compound or composition is administered orally.

4. A pharmaceutical composition for use according to claim 3, in form of a table or capsule.

5. A compound for use according to claim 1 or a pharmaceutical composition for use according to claim 2, wherein said compound or composition is administered parenterally.

6. A compound or pharmaceutical composition for use as claimed in claim 5, wherein said compound or composition is administered by intraveneous administration or continuous infusion.
